(19) Europäisches Patentamt

European Patent Office

Office européen des brevets

(11) **EP 0 517 292 B1**

(12) **FASCICULE DE BREVET EUROPEEN**

(45) Date de publication et mention
de la délivrance du brevet:
**02.02.2000 Bulletin 2000/05**

(51) Int. Cl.[7]: **C12N 15/86**, C12N 15/50,
C12N 15/30, C12N 15/40,
A61K 39/295

(21) Numéro de dépôt: **92201406.3**

(22) Date de dépôt: **19.05.1992**

(54) **Virus de l'Avipox recombinant**

Rekombinantes Avipoxvirus

Recombinant Avipox virus

(84) Etats contractants désignés:
**AT BE CH DE DK ES FR GB IT LI NL PT**

(30) Priorité: **27.05.1991 BE 9100507**

(43) Date de publication de la demande:
**09.12.1992 Bulletin 1992/50**

(73) Titulaire: **DIMMINACO AG
8134 Adliswil (CH)**

(72) Inventeurs:
• **Thiry, Georges
Eagan Minnesota MN 55123 (US)**
• **Colau, Didier
B-1390 Grez-Doiceau (BE)**
• **De Wannemaeker, Catherine
B-1700 Dilbeek (BE)**
• **Malarme, Daniel
B-1650 Beersel (BE)**

(74) Mandataire:
**Mannion, Sally Kim, Dr. et al
c/o Wyeth Laboratories,
Huntercombe Lane South,
Taplow
Maidenhead, Berkshire SL6 0PH (GB)**

(56) Documents cités:
EP-A- 0 314 569    EP-A- 0 353 851
WO-A-90/15140

• **J. VIROL. vol. 64, no. 2, 1990, pages 527 - 533; D. SPEHNER ET AL.: 'Construction of Fowlpox Virus Vectors with Intergenic Insertions'**
• **J. VIROL. vol. 64, no. 2, 1990, pages 527 - 533; D. SPEHNER ET AL.: 'Construction of Fowlpox Virus Vectors with Intergenic Insertions'**
• **J. GEN. VIROL. vol. 69, 1988, pages 1025 - 1040; TOMLEY, F. ET AL.: 'Sequence Analysis of an 11.2kb, near-terminal BamH1 Fragment of Fowlpox Virus'**
• **VACCINE vol. 8, 1990, pages 486 - 490; OGAWA, R. ET AL.: 'Recombinant Fowlpox Viruses inducing protective immunity against Newcastle disease and Fowlpox viruses'**
• **J. GEN. VIROL. vol. 71, 1990, pages 621 - 628; BOURSNELL, M. ET AL.: 'Insertion of the fusion gene from Newcastle Disease Virus into a nonessential region in the terminal repeats of fowlpox virus and deminstration of protective immunity induced by the recombinant'**

EP 0 517 292 B1

**Description**

L'invention concerne des virus recombinants dérivés du virus Avipox et en particulier du virus responsable de la variole aviaire, communément appelée Fowlpox. L'invention concerne également un procédé d'obtention de ces virus par culture de cellule, l'utilisation de ces virus pour l'obtention de vaccins et des vaccins contenant ces virus. L'invention concerne également les séquences de transfert pour l'insertion dans le génome de ce virus recombinant ainsi que les plasmides qui portent ces séquences.

Le virus responsable de la variole aviaire, ou Fowlpox virus (FPV), appartient au genre Avipox de la famille des Poxviridae.

Le virus Fowlpox a les caractéristiques typiques des Pox. Ce sont des virus de grande taille dont le génome est de l'ADN double brin linéaire d'environ 300 kb et dont les extrémités simple brin sont liées de manière covalente. Environ 20 kb du génome ont été séquencés, en particulier la séquence des répétitions terminales inverses (TIR pour Terminal Inverted Repeats) (Campbell et al, 1989; Tomley et al, 1988). Les répétitions terminales inverses sont deux longues séquences identiques présentes à chaque extrémité du génome en orientation inverse.

Le virus de la vaccine ou Vaccinia a été le premier Pox développé comme virus recombinant vivant capable d'exprimer des protéines étrangères. La liste des protéines exprimées est très longue. En particulier, l'expression de nombreux antigènes étrangers a permis d'obtenir des immunisations contre différentes maladies.

On a proposé dans la demande de brevet européen 0 314 569 l'utilisation du virus du Fowlpox à titre de vecteur d'expression de protéines hétérologues en vue de préparer un vaccin, c'est-à-dire l'utilisation d'un virus recombinant ayant intégré dans une région intergénique non codante de son génome une séquence d'ADN codant pour une protéine hétérologue; cette région est une courte séquence de 32 nucléotides, située entre les phases ouvertes de lecture (ORF pour Open Reading Frame) ORF 7 et ORF 9, cette région a été aménagée génétiquement en site d'insertion non essentiel. Dans des régions intergéniques aussi courtes, le risque est grand que l'insertion sépare un signal de transcription de son gène et par conséquent introduise une mutation qui inactive ce gène. Ceci se vérifie : en effet cette séquence de 32 nucléotides semble être une région essentielle, puisqu'elle a dû être élargie à 55 nucléotides en vue d'éviter le recouvrement possible entre le promoteur de l'ORF 9 et la séquence codante 3' de l'ORF 7. Dans le cas de la région qui sépare l'ORF 7 de l'ORF 9, l'insertion dans la séquence naturelle est par ailleurs instable (Spehner et al, 1990), et son aménagement est indispensable. De plus la manière d'aménager une courte région intergénique proposée n'est pas générale.

Par ailleurs, on a également proposé dans la demande de brevet international WO88/02022 la construction de virus du Fowlpox, et autres pox aviaires, recombinants caractérisés par l'insertion d'ADN étranger dans le gène de la thymidine kinase. Cette insertion crée une mutation dans le gène qui pourrait atténuer le pouvoir infectieux du virus. Ceci présente le risque d'une trop grande atténuation de la souche et un pouvoir immunogène réduit du vaccin dérivé du virus recombinant.

Par ailleurs, on a également proposé dans la demande de brevet européen 0 353 851 l'utilisation d'un virus recombinant du Fowlpox à titre de vecteur d'expression de protéine hétérologue. L'insertion du gène étranger dans le génome viral est effectuée dans des phases ouvertes de lecture (ORF) situées dans les répétitions terminales inverses (TIR). L'insertion d'un ou plusieurs gènes étrangers à l'intérieur de phases ouvertes de lecture présente le risque de muter un gène dont on ignore la fonction.

La présente invention fournit d'autres régions d'insertion d'un ou plusieurs gènes étrangers dans le génome viral, qui ne présentent plus les inconvénients des régions d'insertions précitées.

La présente invention concerne à cet effet un virus de l'Avipox recombinant dérivé d'une souche atténuée de virus de l'Avipox et comportant, dans une partie non essentielle de son génome, au moins une séquence d'ADN codant pour tout ou partie d'une protéine hétérologue au virus de l'Avipox, ainsi que les éléments susceptibles d'assurer l'expression de cette protéine dans une cellule infectée par ledit virus recombinant, caractérisé en ce que la partie non essentielle du génome est constituée par une région intergénique non codante, ayant une séquence de plus de 60 nucléotides et située entre deux phases ouvertes de lecture (ORF), leur signaux d'expression y compris, cette région intergénique étant choisie parmi la région, dite région β1, située entre les ORF1 et ORF2 de la région TIR (répétitions terminales inverse) et la région, dite région β2, située entre les ORF2 et ORF3 de la région TIR.

Par une partie non essentielle du génome du virus de l'Avipox, on entend une région qui peut être modifiée sans altérer des fonctions impliquées dans le développement du virus in vitro et in vivo. On choisit comme partie non essentielle une région intergénique non codante du génome, c'est-à-dire située entre deux ORF, leurs signaux d'expression y compris.

Les régions intergéniques selon l'invention sont larges, de sorte que le risque qu'une insertion sépare les signaux de transcription de leurs gènes respectifs soit minime et également que leur aménagement soit inutile. D'autre part, les séquences de ces régions intergéniques sont non codantes et donc le risque de mutation dans une ORF est nul. Par région large on entend une région ayant une séquence de plus de 60 nucléotides. Généralement on choisit une région ayant une séquence de plus de 100 nucléotides. De préférence on choisit une région ayant une séquence de plus de 200 nucléotides. De manière particulièrement préférée on choisit une région ayant une séquence de plus de 400 nucléotides.

Par ailleurs, généralement, on choisit une région intergénique, dans laquelle au moins une séquence d'ADN est clonée, située dans l'une des deux régions TIR du virus de l'Avipox. Habituellement au moins une séquence d'ADN est clonée dans au moins l'une des deux régions TIR du virus de l'Avipox. De préférence au moins une séquence d'ADN est clonée dans les deux régions TIR du virus. De manière encore préférée on choisit la région intergénique, dite région β1, ou/et la région intergénique, dite région β2; la région intergénique, dite région β1, étant située entre les nucléotides 1675 et 2165 et la région intergénique, dite région β2, étant située entre les nucléotides 2672 et 3605 en prenant comme nucléotide d'origine le site de restriction BamH1 présent dans les TIR. Ce site de restriction <u>Bam</u>H1 choisi ici pour positionner les régions intergéniques est le premier nucléotide de la séquence publiée par Tomley et al, 1988. On peut également définir ces régions selon l'invention comme suit : la région intergénique, dite région β1, est située entre les phases ouvertes de lecture ORF 1 et ORF 2, la phase ouverte de lecture ORF 1 étant située entre les nucléotides 416 -le nucléotide A de l'ATG- et 1674 -le 3ème nucléotide du codon stop- et la phase ouverte de lecture ORF 2 étant située entre les nucléotides 2166 -le nucléotide A de l'ATG- et 2671 -le 3ème nucléotide du codon stop-, en prenant comme nucléotide d'origine le site de restriction <u>Bam</u>H1 dans les TIR et la région intergénique, dite région β2, est située entre les phases ouvertes de lecture ORF 2 et ORF 3, la phase ouverte de lecture ORF 2 étant située entre les nucléotides 2166 -le nucléotide A de l'ATG- et 2671 -le 3ème nucléotide du codon stop- et la phase ouverte de lecture ORF 3 étant située entre les nucléotides 3606 -le 3ème nucléotide du codon stop- et 4055 -le nucléotide A de l'ATG-, en prenant comme nucléotide d'origine le site de restriction <u>Bam</u>H1 dans les TIR. De manière particulièrement préférée on choisit dans la région β1 une partie située entre les nucléotides 1775 et 2065 et dans la région β2 une partie située entre les nucléotides 2772 et 3505. De bons résultats ont été obtenus en clonant au niveau du nucléotide 1842, dit site d'insertion B1, situé dans la région β1, et/ou au niveau du nucléotide 3062, dit site d'insertion B2, dans la région β2.

De bons résultats ont été obtenus lorsqu'au moins une séquence d'ADN est clonée dans la région β2, dans l'une des deux régions TIR du virus de l'Avipox. De bons résultats ont été également obtenus lorsqu'une séquence d'ADN est clonée dans la région β1 dans chacune des deux TIR. Cela représente un autre avantage de l'invention puisqu' il est possible d'obtenir deux copies de la séquence d'ADN hétérologue par génome.

Par virus de l'Avipox recombinant dérivé d'une souche atténuée de virus de l'Avipox, on entend un virus appartenant au genre Avipox, atténué, dont le génome comprend une séquence hétérologue accompagnée de signaux d'expression. Généralement on utilise comme virus un virus du genre Avipox tel que le Fowlpox, le Pigeonpox ou le Canarypox. Habituellement on utilise comme virus le Fowlpox. De préférence, on utilise comme virus Fowlpox une souche vaccinale du virus de la variole des poules ou Fowlpox tel que le vaccin produit et commercialisé par la Société SOLVAY sous la marque POXINE ou le vaccin produit et commercialisé par la Société SOLVAY sous les marques de CHICK-N-POX, POULVAC CHICK-N-POX, CHICK-N-POX TC, POULVAC CHICK-N-POX TC, POULVAC POXINE, POULVAC P. De manière particulièrement préférée on utilise comme virus du Fowlpox le vaccin commercialisé par SOLVAY sous les marques de CHICK-N-POX, POULVAC CHICK-N-POX, CHICK-N-POX TC, POULVAC CHICK-N-POX TC, POULVAC POXINE, POULVAC P, en abrégé CNP.

L'atténuation est obtenue par des passages successifs sur embryon, ou si le virus a été adapté à des cellules en culture, par des passages sur culture de cellules.

Par éléments susceptibles d'assurer l'expression du gène qui code pour la protéine hétérologue dans une cellule infectée par ledit virus recombinant, on entend les signaux d'expression génétique reconnus par le virus, notamment le promoteur et le terminateur, éléments connus de l'homme du métier et tel que notamment décrits par exemple par Moss, 1990. Généralement on utilise un promoteur de Pox. Habituellement on utilise un promoteur de Vaccinia ou de Fowlpox. De préférence on utilise le promoteur P11 et le promoteur P7.5 de Vaccinia, tels que décrits par Venkatesan et al, 1981, Cochran et al, 1985 et Bertholet et al, 1985.

Par au moins une séquence d'ADN codant pour tout ou partie d'une protéine hétérologue au virus de l'Avipox, on entend tout fragment d'ADN extrait d'un génome, ou une copie ADNc ou encore un ADN de synthèse, accompagné des signaux d'expression, et dont la séquence code pour tout ou partie d'une protéine étrangère à l'Avipox.

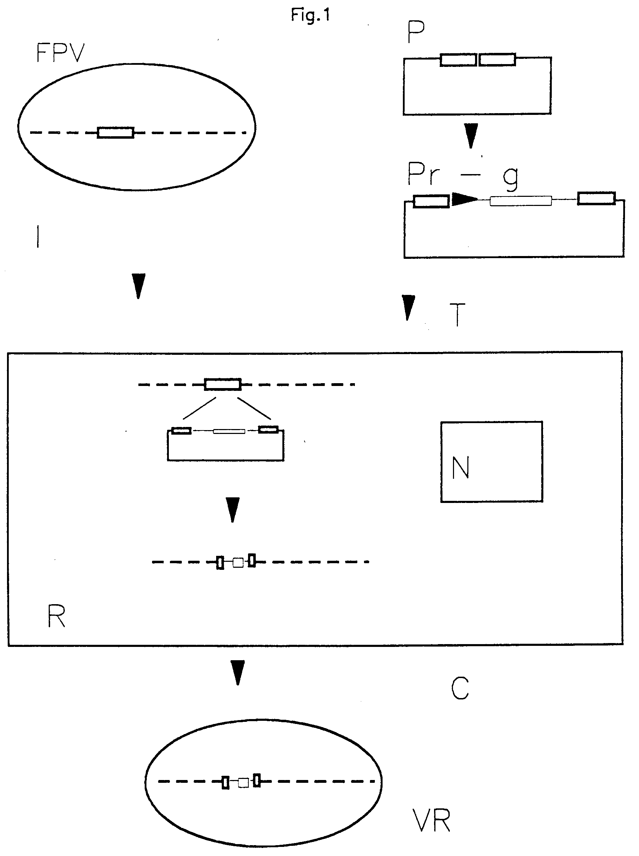

Les figures 1 à 11 ont été réalisées pour mieux permettre la compréhension de l'invention.

La figure 1 représente une construction d'un Avipox recombinant, modifié d'après Mackett et al, 1984. Des cellules infectées (I : infection) par le virus du Fowlpox (FPV) sont transfectées (T : "transfection") par un plasmide de transfert. Ce plasmide (P) porte un promoteur (Pr) orienté pour permettre l'expression d'un gène (g) hétérologue adjacent, flanqué de part et d'autre par des séquences d'ADN viral. La recombinaison (R) homologue a lieu dans le cytoplasme de la cellule (C), entre les séquences qui flanquent le gène et les séquences présentes sur le génome viral. Il en résulte l'insertion du gène hétérologue dans le génome viral. Ce génome peut être empaqueté et produire des particules virales recombinantes (VR). N pour noyau de la cellule (C).

La figure 2 représente schématiquement les extrémités du génome du Fowlpox avec les TIR de droite (TIR-R) et de gauche (TIR-L) symbolisés par des rectangles et avec la séquence unique symbolisée par un trait. Les pointillés symbolisent la région centrale du génome. Le fragment de restriction EcoR1 du TIR-L est de 6.2 kb tandis que celui de TIR-R est de 9.0 kb. Ce schéma montre que ces deux fragments ont une séquence commune et une séquence unique. Un site BamH1, situé en aval de EcoR1 dans les TIR est indiqué.

La figure 3 représente une carte de restriction du plasmide pTIRB1 (PTIRB1) pour quelques sites uniques et la position des ORF. Le site unique BamH1 est utilisé pour les clonages de gènes hétérologues.

    ori (ORI) : origine de réplication du plasmide dans Escherichia coli
    (E. coli); Ap : gène de résistance à l'ampicilline; ORF1 à
    ORF6 : phases ouvertes de lecture du Fowlpox;

La figure 4 représente une figure semblable à la figure 3 mais pour le plasmide pTIRB2 (PTIRB2).

La figure 5 représente une carte de restriction du plasmide p11Lac (P11LAC). Ce plasmide porte le gène LacZ de E. coli sous le contrôle du promoteur P11 de Vaccinia. P11 : promoteur P11 de vaccinia; LACZ : gène codant pour la β-galactosidase; ori : origine de réplication dans E. coli; Brin + : brin empaqueté dans le phage M13; Ap : gène de résistance à l'ampicilline.

La figure 6 représente un exemple d'un vecteur de transfert du gène LacZ dans les TIR. Ce vecteur est le pTIRB1P75Lac (PTIRB1P75LAC). La cassette P7.5-LacZ est clonée dans le site BamH1 du pTIRB1, dans le sens horlogique. P7.5 : promoteur P7.5 de vaccinia.

La figure 7 est une représentation schématique des insertions du gène LacZ (représenté par un rectangle hachuré) dans le génome du CNP. Les cassettes P7.5-LacZ et P11-LacZ sont insérées dans les régions terminales (TIR), dans l'une ou l'autre orientation. La région TIR de droite n'est pas représentée. Les flèches indiquent le sens de transcription des gènes, représentés par des rectangles. L'échelle n'est pas respectée.

La figure 8 représente le segment A de l'IBDV, la position des primers et les différents fragments obtenus par transcription inverse et amplification de l'ARN du segment A de la souche Edgar.

A         : organisation des ORF.
p         : "primers" utilisés pour réaliser l'amplification du segment A.
>,<      : orientation des "primers" par rapport à la séquence codante du segment A.
fgt PCR    : fragments générés par amplification de l'ARN du segment A.
pb        : paires de bases.

La figure 9 (figure 9a à figure 9e) représente la séquence nucléotidique de l'ADN amplifié correspondant à l'ARN du segment A de la souche Edgar et la traduction en acides aminés des ORF 1, 2 et 3.

Primers 0, 1, 1b, 2, 3, 4, 5 et 6 : primers utilisés pour générer, par transcription inverse et amplification, les fragments d'ADN correspondant au segment A de la souche Edgar.

La figure 10 représente le schéma de reconstruction du segment d'ADN correspondant au segment A de la souche Edgar à partir des fragments obtenus par transcription inverse et amplification.

\*        : mutagénèse dirigée.

La figure 11 représente une carte de restriction du plasmide pTIR75E1LAC (PTIR75E1LAC).

La portion du plasmide en trait gras correspond aux séquences homologues au génome du FPV. ori (ORI) : origine de réplication du plasmide dans E. coli; Ap: gène de résistance à l'ampicilline; P7.5: promoteur P7.5 de vaccinia; ORF1-IBDV : ORF1 du segment A de la souche Edgar; P11: promoteur P11 de vaccinia; LACZ: gène codant pour la β-galactosidase; pb: paires de bases; ORF1 à ORF6 : phases ouvertes de lecture du Fowlpox.

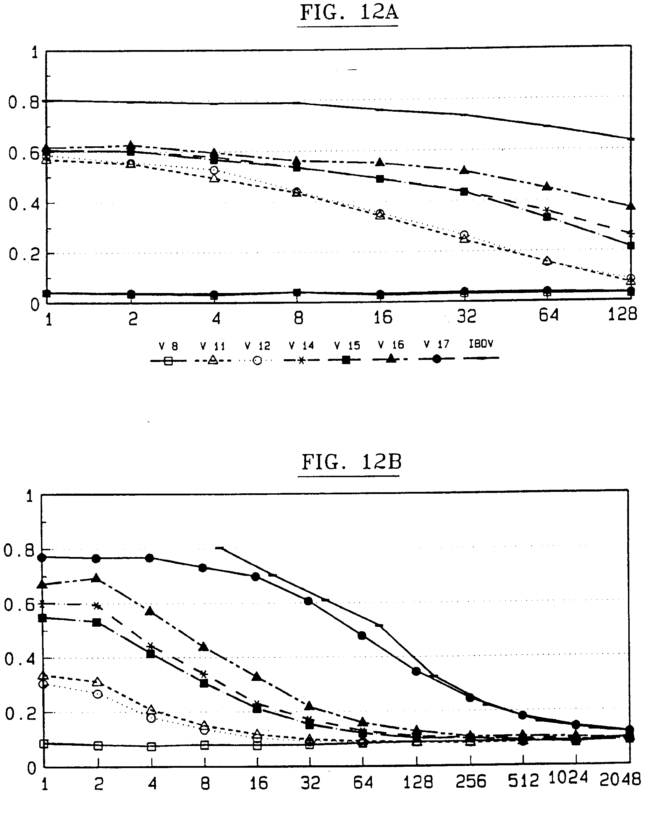

La figure 12 représente la caractérisation par test ELISA des protéines IBDV exprimées dans les recombinants FPV/IBDV. La caractérisation a été réalisée comme suit : 50 μl des suspensions cellulaires et des dilutions en série de deux de ces suspensions ont été déposés par cupule. Après incubation du deuxième anti-corps anti-IBDV (monoclonal anti-VP3 ou anti-VP2), une amplification du signal a été réalisée à l'aide du système anti-IgG de souris marqué à la biotine + streptavidine conjuguée à la phosphatase alcaline.

La figure 12 A correspond à la détection à l'aide de l'anticorps monoclonal anti-VP3. La figure 12 B correspond à la détection à l'aide de l'anticorps monoclonal anti-VP2.

Dans ces figures l'abscisse représente les dilutions en série de 2 des extraits cellulaires en unités logarithmiques et l'ordonnée représente le rapport des absorbances mesurées à 690 mm et 405 mm.

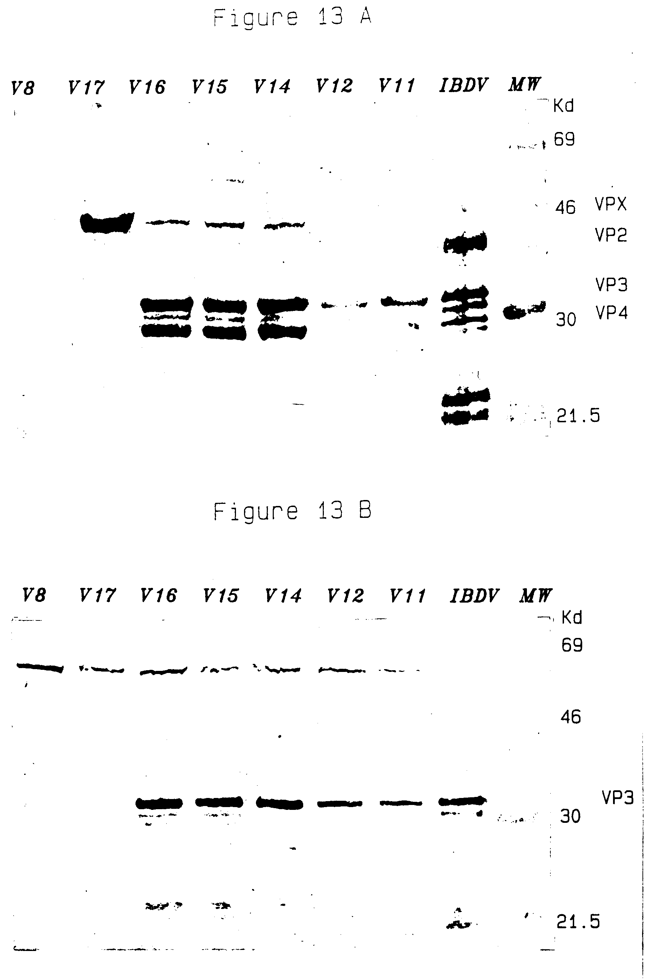

La figure 13 représente la caractérisation par Western blot des protéines IBDV exprimées dans les recombinants FPV/IBDV. Cette caractérisation a été réalisée comme suit : les suspensions cellulaires sont centrifugées à 3000 g et les surnageants obtenus ultracentrifugés à 185000 g; les culots résultants des deux centrifugations sont rassemblés et remis en suspension dans un tampon PBS (Phosphate Buffered Saline, Gibco).

Un dépôt de protéines de 20 à 40 μg pour les recombinants FPV/IBDV et 5 μg pour les cellules infectées par l'IBDV (contrôle positif) est déposé par puit, sur gel acrylamide-SDS. Après transfert sur membrane, les protéines IBDV sont détectées à l'aide du sérum polyclonal reconnaissant toutes les protéines IBDV pour la figure 13 A ou à l'aide de l'anticorps monoclonal anti-VP3 pour la figure 13 B. Une amplification du signal est réalisée à l'aide du système anti-IgG marqué à la biotine et le conjugué streptavidine-phosphatase alcaline. PM : protéines repères de poids moléculaires.

Le procédé mis en oeuvre pour réaliser l'invention est schématisé à la figure 1 (modifié d'après Mackett et al, 1984). Des cellules infectées par l'Avipox ou le Fowlpox sont transfectées par un plasmide de transfert. Ce plasmide porte un promoteur bien orienté pour permettre l'expression d'un gène hétérologue adjacent, flanqué de part et d'autre par des séquences d'ADN viral. La recombinaison homologue a lieu dans le cytoplasme de la cellule entre les séquences qui flanquent le gène et les séquences présentes sur le génome viral. Il en résulte l'insertion du gène hétérologue dans le génome viral. Ce génome peut être empaqueté et produire des particules virales recombinantes. Cette technique d'insertion par infection des cellules par le Pox suivie d'un transfert par un plasmide a été mise au point pour Vaccinia, et couramment utilisée dans de nombreux laboratoires pour la construction de Pox recombinants (Mackett et al, 1985).

Le procédé selon l'invention comprend habituellement les étapes suivantes :

- la première étape consiste à construire les plasmides de transfert autorisant le criblage,
- la deuxième étape consiste à cloner les gènes codant pour les antigènes hétérologues dans les plasmides de transfert,
- la troisième étape consiste à construire les virus Avipox recombinants, et
- la quatrième étape consiste à effectuer les tests de vaccination avec les virus recombinants.

De préférence le procédé comprend les étapes suivantes :

a. Construction des plasmides de transfert autorisant le criblage :

- Clonage des fragments portant TIR.
- Insertion de site de clonage dans les TIR.
- Clonage des promoteurs P11 et P7.5 de Vaccinia.
- Construction d'une cassette portant le gène LacZ et son clonage en aval de P11 ou de P7.5.
- Clonage des éléments P11LacZ ou P7.5LacZ dans les plasmides de transfert TIR.

b. Construction des virus CNP recombinants (gène LacZ pour effectuer un test préliminaire) :

- Transfert des cellules QT35 par le virus CNP et un plasmide de transfert.
- Criblage puis purification des recombinants grâce à l'expression du gène LacZ.
- Analyse des génomes des recombinants et de l'expression de LacZ.

c. Test de vaccination :

- Vaccination de poussins par des virus recombinants et protection contre la variole aviaire conférée par le virus recombinant LacZ.

d. Clonage de gènes codant pour des antigènes hétérologues (protéine hétérologue) dans les plasmides de transfert :

- Clonage des gènes codant pour des antigènes hétérologues en aval de P11 ou de P7.5.
- Clonage des cassettes P11-Antigène et P7.5-LacZ ou P7.5-Antigène et P11-LacZ dans les plasmides TIR.

e. Construction des virus CNP recombinants :

- Transfert des cellules QT35 par le virus CNP et un plasmide de transfert.
- Criblage puis purification des virus recombinants grâce à l'expression du gène LacZ.
- Analyse de l'expression de l'antigène sur culture de cellules et analyse du génome des virus recombinants.

f. Vaccination :

- Vaccination par les virus CNP recombinants.
- Evaluation du degré de protection conféré par l'expression de l'antigène hétérologue au CNP.

L'invention concerne également des séquences de transfert qui permettent d'insérer l'ADN hétérologue dans le génome du virus de l'Avipox, tel que défini ci-avant et notamment aux sites B1 ou B2. L'invention concerne également les plasmides qui portent ces séquences. Par plasmide de transfert, on entend un plasmide qui comporte un gène hétérologue sous le contrôle d'un promoteur reconnu par l'Avipox, flanqué de part et d'autre par les séquences situées de part et d'autre du site B1 ou du site B2. La longueur de ces séquences homologues au FPV qui flanquent le gêne hétérologue doivent être suffisamment longues pour autoriser une recombinaison à gauche et à droite du gène hétérologue. Généralement on choisit des séquences d'au moins 1 kb. De bons résultats sont obtenus pour B1 flanqué d'une séquence d'environ 1850 nucléotides en amont et 4321 nucléotides en aval, et, pour B2, environ 3070 nucléotides en amont et 3100 nucléotides en aval. Les plasmides de transfert interviennent lors du procédé pour isoler des Avipox recombinants, le procédé faisant intervenir un transfert des cellules de caille QT35, préalablement infectées par le virus de l'Avipox.

L'invention concerne également une culture de cellules eucaryotes infectées par un virus de l'Avipox recombinant dérivé d'une souche atténuée de virus d'Avipox et comportant dans une partie non essentielle de son génome au moins une séquence d'ADN codant pour tout ou partie d'une protéine hétérologue au virus de l'Avipox ainsi que les éléments susceptibles d'assurer l'expression de cette protéine dans une cellule infectée par ledit virus recombinant, la partie non essentielle étant constituée par une région intergénique non codante du virus de l'Avipox, située entre deux ORF, leurs signaux d'expression y compris.

De préférence on utilise une culture de cellules aviaires. De bons résultats ont été obtenus avec une culture de cellules de caille. D'excellents résultats ont été obtenus avec la culture de cellules de caille QT35, telle que décrite par Cho (1981).

Le virus de l'Avipox recombinant selon l'invention est développé comme vecteurs capables d'exprimer tout ou partie de la protéine hétérologue. La présente invention concerne donc également l'utilisation du virus selon l'invention comme vecteur capable d'exprimer tout ou partie d'une protéine hétérologue, telle que notamment un antigène.

L'invention concerne également un vaccin contenant un virus de l'Avipox recombinant dérivé d'une souche atténuée de virus d'Avipox et comportant dans une partie non essentielle de son génome au moins une séquence d'ADN codant pour tout ou partie d'une protéine hétérologue au virus de l'Avipox ainsi que les éléments susceptibles d'assurer l'expression de cette protéine dans une cellule infectée par ledit virus recombinant, la partie non essentielle du génome étant constituée par une région intergénique non codante du virus de l'Avipox, située entre deux ORF, leurs signaux d'expression y compris.

Par protéine hétérologue, on entend toute protéine ou partie de protéine étrangère au virus d'Avipox, protéine naturelle ou modifiée. Généralement, cette protéine peut être un antigène permettant de développer un vaccin contre un organisme pathogène. Habituellement les antigènes sont notamment les protéines d'agents infectieux aviaires, virus, bactéries, chlamydies, mycoplasmes, protozoaires, champignons et vers.

Ce sont notamment les Adénoviridae, tels que le virus responsable de l'anémie aplastique (AA) et du syndrome de la chute de ponte (EDS), les Birnaviridae, tel que le virus de la maladie de Gumboro (IBDV), les Coronaviridae, tel que le virus de la bronchite infectieuse (IBV), les Herpesviridae, tels que le virus de la maladie de Marek (MDV) et le virus responsable de la laryngotrachéite infectieuse (ILTV), les Orthomyxoviridae, tel que le virus responsable de la grippe (Influenza), les Paramyxoviridae, tel le virus de la maladie de Newcastle (NDV), les Picornaviridae, tel que le virus responsable de l'encéphalomyélite (AE), les Réoviridae, responsables de ténosynovite, les Rétrovirus tel que le virus responsable de la leukose lymphoïde (LL), le virus responsable de l'anémie du poulet (CAV).

Ce sont notamment des chlamydies, tels que Chlamydia psittaci.

Ce sont notamment des mycoplasmes tels que Mycoplasma gallisepticum et M. synoviae.

Ce sont notamment des bactéries telles que Escherichia coli, Haemophilus dont H. paragallinarum qui est responsable du coryza, Pasteurella dont P. multocida qui est responsable du cholera, Salmonella dont S. gallinarium responsable du typhus et S. pullorum, Clostridium dont C. botulinum qui produit une toxine responsable du botulisme, C. perfringens et C. septicum responsables de dermatoses, C. colinum, Campilobacter jejuni, Streptococcus aureus.

Ce sont notamment des protozoaires tels que Eimeria responsable de la coccidiose et Cryptosporidiosis baileyi responsable de la cryptosporodiose.

Ce sont notamment des vers tels que les cestodes et nématodes dont Ascaridia galli.

Ce sont notamment des champignons tels que Aspergillus fumigatus.

Les antigènes préférés sont ceux du virus de la maladie de Gumboro (IBDV), du virus de la bronchite infectieuse (IBV), du virus responsable de l'anémie du poulet (CAV), du protozoaire Eimeria responsable de la coccidiose, du virus de la maladie de Newcastle (NDV) et du virus de la maladie de Marek (MDV).

Les antigènes particulièrement préférés sont ceux du virus de la maladie de Gumboro (IBDV), du virus de la bronchite infectieuse (IBV), du virus responsable de l'anémie du poulet (CAV) et du protozoaire Eimeria, responsable de la coccidiose. De bons résultats ont été obtenus dans les conditions suivantes :

- pour l'IBDV : tout ou partie des phases de lecture ouvertes dont la polyprotéine et des parties de la polyprotéine. De telles protéines sont notamment VP2, VP3, VP4. Ceci comprend également toutes leurs combinaisons ou modifications. Ces modifications consistent par exemple en l'insertion d'un site de clivage.
- pour l'IBV : l'antigène E2.
- pour Eimeria : l'antigène de surface TA4 naturel et l'antigène de surface TA4 dont la séquence du site de clivage protéolytique a été modifiée.
- pour le CAV : la protéine P50.

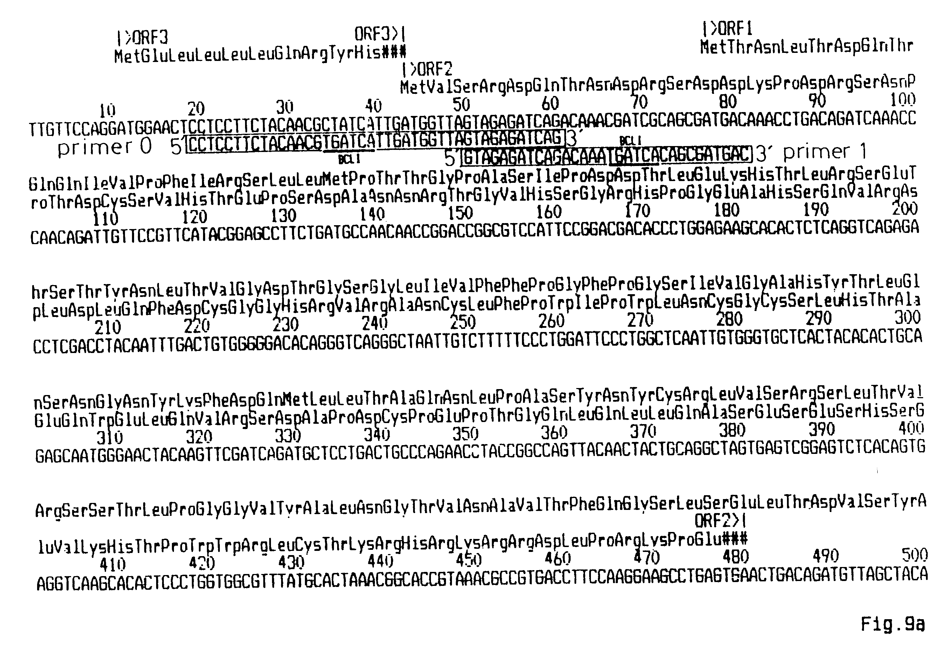

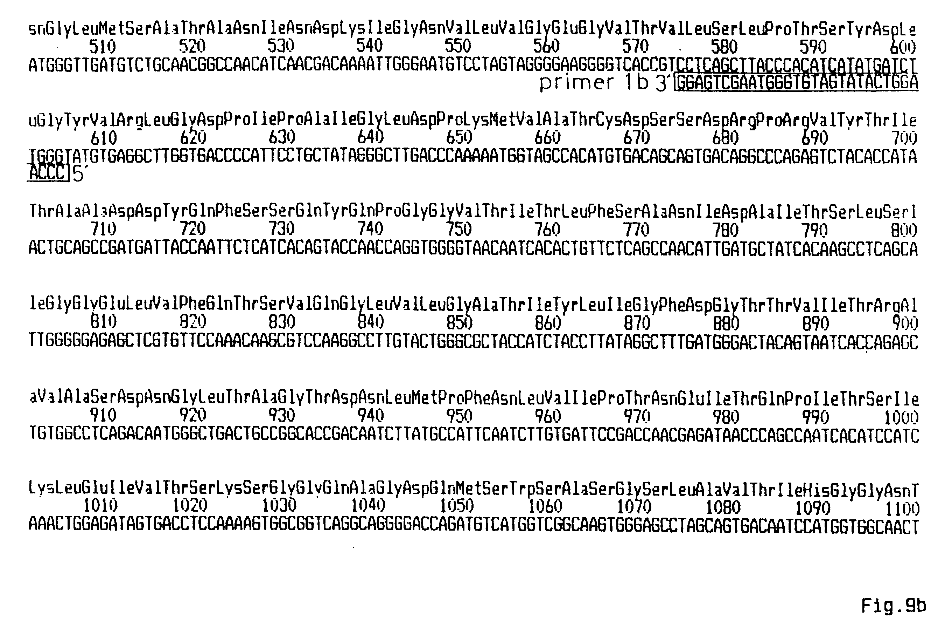

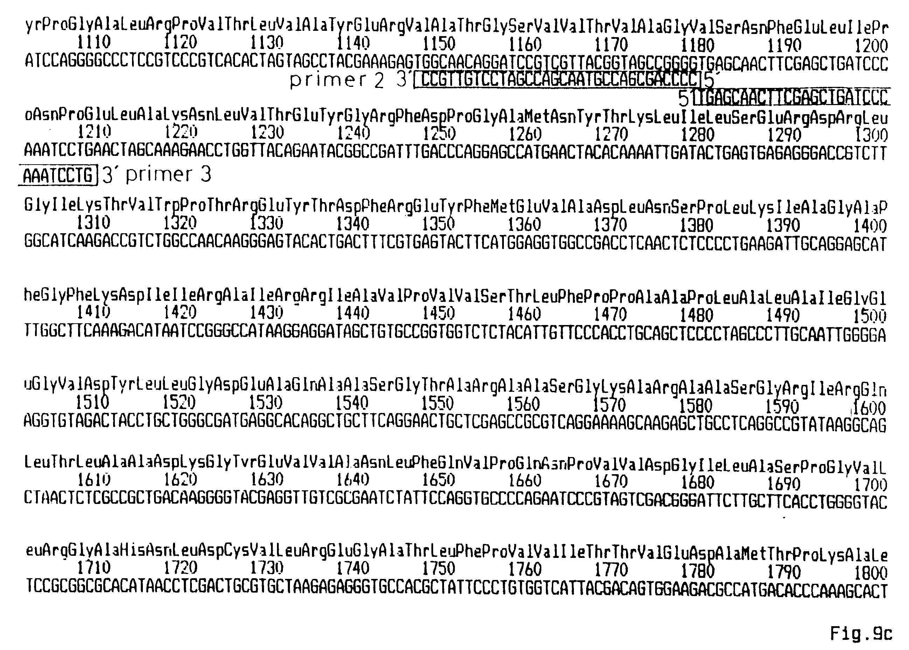

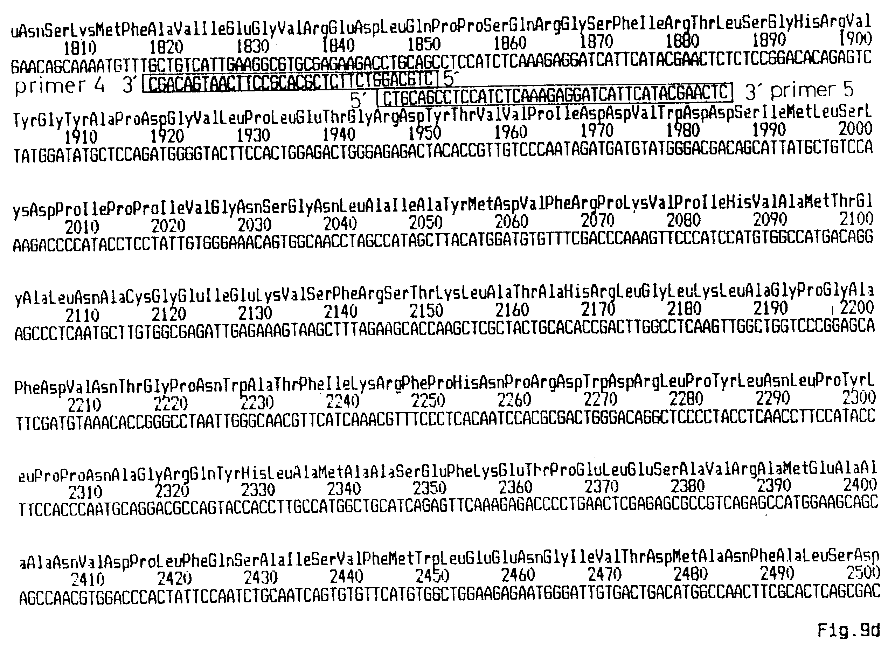

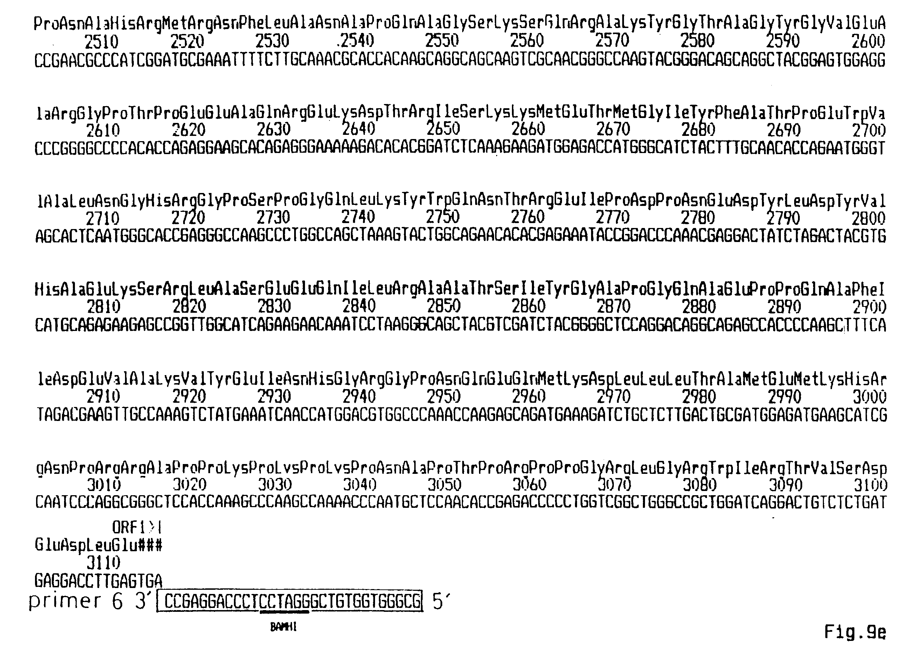

D'excellents résultats ont été obtenus avec l'IBDV, la protéine hétérologue étant une partie de la polyprotéine comprenant les acides aminés 1 à 493 suivis des acides aminés 1010 à 1012, dans laquelle est incluse la protéine VP2; comme représenté à la figure 9.

Les séquences d'ADN peuvent coder pour la protéine au complet ou bien pour des fragments de la protéine, et induire chez l'animal la réponse immunitaire appropriée. D'autres molécules que des antigènes peuvent égalemert être prises en considération dont des facteurs de croissance et des immunomodulateurs, telles que des interleukines. La présente invention vise également à produire in vivo, dans la volaille, un facteur intervenant dans le métabolisme de l'animal, tel que par exemple une hormone de croissance ou un facteur inducteur de celle-ci. La présente invention concerne également la préparation de protéines ou de peptides en culture de cellule in vitro ou in vivo chez l'animal. En fonction de la protéine hétérologue introduite, elle peut notamment convenir comme enzyme, comme constituant nutritif ou dans le domaine de la santé humaine ou animale en tant que produit pharmaceutique à usage humain ou vétérinaire.

D'autre part, des virus recombinants multiples, porteurs de plusieurs gènes hétérologues peuvent être générés, soit en clonant plusieurs gènes dans le même génome, dans un même site ou dans des régions ou des sites différents. Un produit polyvalent peut également être créé en combinant des virus recombinants différents.

Les cibles animales du virus de l'Avipox selon l'invention sont les oiseaux, en particulier la volaille. Le virus recombinant peut être également employé dans des espèces non aviaires.

Le vaccin selon l'invention peut être administré sous diverses formes connues de l'homme du métier. Habituellement le vaccin est administré par voie orale, dans l'alimentation ou dans l'eau de boisson, par voie intranasale, par injection sous-cutanée, par aérosol, par injection intramusculaire ou par transpercement de l'aile selon la méthode dite du "wing web". De préférence le vaccin est administré par transpercement de la membrane alaire selon la méthode dite du "wing web", par injection intramusculaire ou par injection sous-cutanée.

Le vaccin selon l'invention est formulé de manière connue par l'homme du métier. Habituellement la composition vaccinale contient des stabilisants adaptés au mode d'administration du vaccin. Il peut être conservé sous forme lyophilisée.

La présente invention est illustrée par les exemples suivants.

## Exemple 1 - Purification de virus CNP et de son ADN

On utilise comme virus du Fowlpox la souche vaccinale commercialisée par la firme Solvay sous la marque POULVAC CHICK-N-POX, CHICK-N-POX, CHICK-N-POX TC, POULVAC CHICK-N-POX TC, (CNP), comme vaccin vivant atténué. Le stock initial provient d'une souche de terrain passée à plusieurs reprises sur oeufs et adaptée aux cellules in vitro par 2 passages sur CEF (fibroblastes d'embryon de poulet) et 5 passages sur la lignée cellulaire de caille QT35 décrite par Cho (1981). La lignée cellulaire de caille QT35 est accessible auprès du Dr. Moscovici (6816 Northvest 18th Avenue, Gainesville, Florida 32605, Etats-Unis) qui l'a isolée et qui la distribue.

CNP est cultivé sur la lignée cellulaire QT35 dont le milieu de croissance est composé de E199 (Gibco, 500 ml), F12 (Gibco, 500 ml), LAH (Gibco, hydrolysat de lactalbumine, 25 ml), FCS (Gibco, Foetal Calf Serum, 25 ml) et fructose (5 ml d'une solution à 200 g/l); incubation à 38°C, 3 % de $CO_2$. La multiplicité d'infection (moi) habituelle est de 0,01 virus par cellule, les cellules étant à 80 % de la confluence.

Un stock de virus a été purifié comme suit : les cellules infectées par le virus du Fowlpox CNP et reprises dans du tampon phosphate PBS (Phosphate Buffered Saline, Gibco) sont centrifugées à 6.000 g (15 min). Le culot est remis en suspension dans du Tris (pH 9, 1 mM), trypsinisé (trypsine à 0.25 mg/ml concentration finale; Gibco) et soniqué. Ce matériel est déposé sur un coussin de sucrose 36 % (poids/volume) et centrifugé 45 minutes à 30.000 g. Le culot est repris dans du Tris 1 mM, pH 9.0. Les virus sont centrifugés une dernière fois 30 minutes à 40.000 g. Le culot est repris dans le tampon de lyse : Tris-HCl 10 mM, pH 8.0, EDTA 10 mM, SDS (Sodium Dodecyl Sulfate) 1 %, protéinase K 500 μg/ml (Boehringer), RNase 0.1 mg/ml (Boehringer) et incubé 2 heures à 37°C. L'ADN extrait au phénol est précipité à l'éthanol. De l'ordre de 20 μg d'ADN ont été purifiés à partir de 40 flasques de 150 cm$^2$ infectées.

## Exemple 2 - Construction d'une librairie génomique EcoR1 et une librairie génomique BamH1 du CNP

Les techniques de génétique utilisées sont décrites par Maniatis et al, 1982. Les enzymes de restriction, polymérases, ligases et phosphatases ont été fournis par les firmes Pharmacia, Boehringer et Biolabs. Les ADN synthétiques ont été fournis par la firme Eurogentec.

Les fragments de restriction de l'ADN viral par EcoR1 ou BamH1 ont été ligaturés dans le vecteur pUC18 décrit par Messing (1983) et introduits dans la bactérie E. coli MC1061 (araD139, (ara, leu)7697, lacX74, galU, galK, hsdR, strA, vendue par la Société Pharmacia). Les deux banques génomiques sont conservées à -70°C dans du glycerol sous forme de deux suspensions de cellules transformées. Quatre-vingts % des plasmides contiennent un insert. Dix-sept fragments BamH1 et 45 fragments EcoR1 furent différenciés sur base de leur taille et leur modèle de restriction par Bg12 ou HinF1. Les inserts ont des tailles de 0,5 à 15 kb; la somme des inserts BamH1 ainsi caractérisés est de 82 kb et de 210 kb pour les fragments EcoR1. La taille du génome du Pox aviaire est proche de 300 kb (Coupar et al, 1990). Les fragments isolés représentent dès lors 25 % pour BamH1 et 70 % pour EcoR1 du génome total.

## Exemple 3 - Clonage des fragments portant les TIR

Les répétitions terminales inverses (TIR) sont deux séquences identiques présentes aux extrémités du génome des Pox en orientation opposée. Une séquence de plus de 17 kb d'une extrémité du génome de la souche HP du Fowlpox a été publiée (Campbell et al, 1989 et Tomley et al, 1988). Elle comprend environ 10 kb de la séquence répétée et 7.0 kb de sa séquence adjacente.

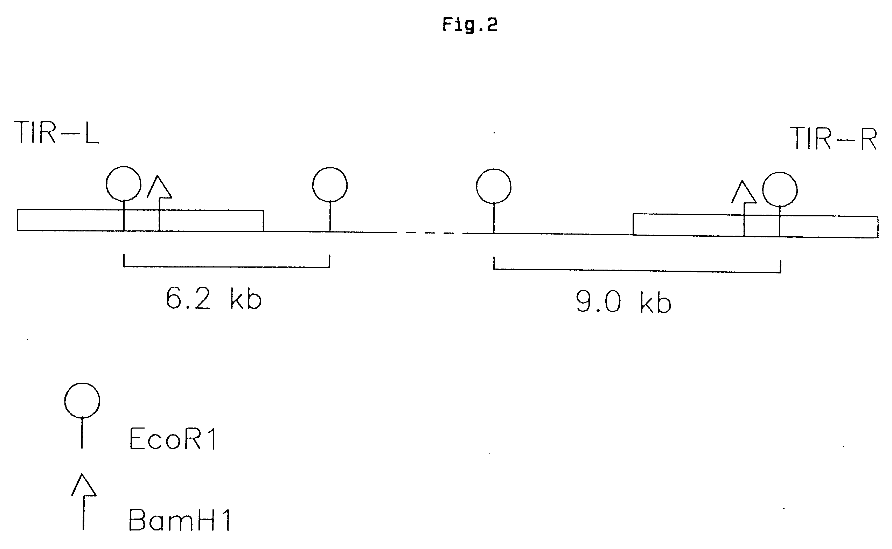

On effectue le clonage des fragments EcoR1 des TIR. La séquence répétée contient un site EcoR1. Comme le montre la figure 2, deux fragments EcoR1 sont clonés : chacun porte une partie de TIR et une séquence adjacente unique. La séquence unique est délimitée par le site EcoR1 le plus proche des TIR. Ces deux fragments ont été isolés grâce à un oligonucléotide complémentaire à la séquence située dans la partie répétée. Le premier fragment a une taille de 6.2 kb et sera, par convention, noté TIR-L. Le second a une taille de 9.0 kb et sera, par convention, noté TIR-R. La séquence du fragment EcoRI TIR-L de CNP est identique à celle publiée.

La jonction entre le TIR et la séquence unique déduite de la comparaison des séquences de TIR-L et de TIR-R se situe au nucléotide 4007, le site BamH1 de la séquence publiée par Tomley et al (1988) étant pris comme nucléotide numéro 1. La jonction est située dans la phase de lecture ouverte notée ORF3 par ces auteurs.

## Exemple 4 - Création par mutagénèse dirigée de sites BamH1 dans TIR

Des phases ouvertes de lecture (ORF) sont déduites de l'analyse des séquences en nucléotides des TIR. En particulier, ORF 1 est située entre les nucléotides 416 et 1674, ORF 2 est située entre les nucléotides 2166 et 2671 et enfin ORF 3 est située entre les nucléotides 3606 et 4055 et codée sur le brin complémentaire. Nous prenons comme nucléotide n° 1 le premier nucléotide G du site BamH1 des TIR. Ces ORF délimitent deux larges séquences intergéniques non codantes, notées région β1, entre ORF1 et ORF2 d'une part, et région β2 entre ORF2 et ORF3 d'autre part. Ce sont ces deux régions intergéniques qui sont choisies comme régions d'insertion.

Un site de clonage unique a été introduit par mutagénèse dirigée (kit de mutagénèse BioRad) dans β1 et dans β2. Le site BamH1 a été choisi comme site de clonage parce qu'il est compatible avec d'autres sites de restrictions, dont Bcl1 et Bgl2. L'emplacement du site de clonage a été choisi environ au milieu de chaque région intergénique pour éviter tout risque de séparer les signaux de transcription de leurs gènes respectifs, soit :

- dans la région β1 : le site B1 est la séquence 5'GATC 3', au nucléotide 1842, transformée en BamH1 GGATCC par insertion d'un G en 5' et d'un C en 3';
- dans la région β2 : le site B2 est la séquence 5'GGATT 3', au nucléotide 3062, transformée en BamH1 par la mutation du second T en CC.

Il y a deux sites BamH1 dans le plasmide qui porte le fragment TIR-L (exemple 3). Un site provient du vecteur pUC18. Le second site provient de TIR et est situé à 71 nucléotides en aval du site EcoR1 choisi pour le clonage (Campbell et al, 1989; figure 2). Ces deux sites BamH1 ont été délétés du plasmide porteur de TIR-L par une restriction BamH1 suivie du remplissage à la polymérase de Klenow et ligation. Le plasmide qui en résulte est le pTIR1.

Pour générer un site BamH1 en B1, le fragment HindII de 0.9 kb de pTIR1 a été cloné dans le vecteur pBSPlus commercialisé par la firme Stratagene. Le primer de mutagénèse fut : 5'TTTCGAAAGACTTT**GGATCC**GTAGTATAATATTATA 3'. Les nucléotides en gras, c'est-à-dire GGATCC, sont la séquence reconnue par BamH1.

Pour générer un site BamH1 en B2, le fragment XbaI de 1.7 kb du pTIR1 a été cloné dans pBSPlus. Le primer fut : 5'TATATCACG**GGATCC**AAAAGGTTATTAGTAGTC 3'. Les nucléotides en gras, c'est-à-dire GGATCC, sont la séquence reconnue par BamH1.

La réinsertion de chaque fragment muté dans pTIR1 est le résultat des ligations :

- pour B1 : Hind3-Sca1 du pTIR1 (1491 bp) + Sca1-Aat2 du pBSPlus après mutagénèse (485 bp) + Aat2-EcoR1 du pTIR1 (4220 bp) + EcoR1-Hind3 du pTIR1 (2635 bp);
- pour B2 : Xho1-Spl1 du pTIR1 (8512 bp) + Spl1-Xho1 du pBSPlus après mutagénèse (318 bp).

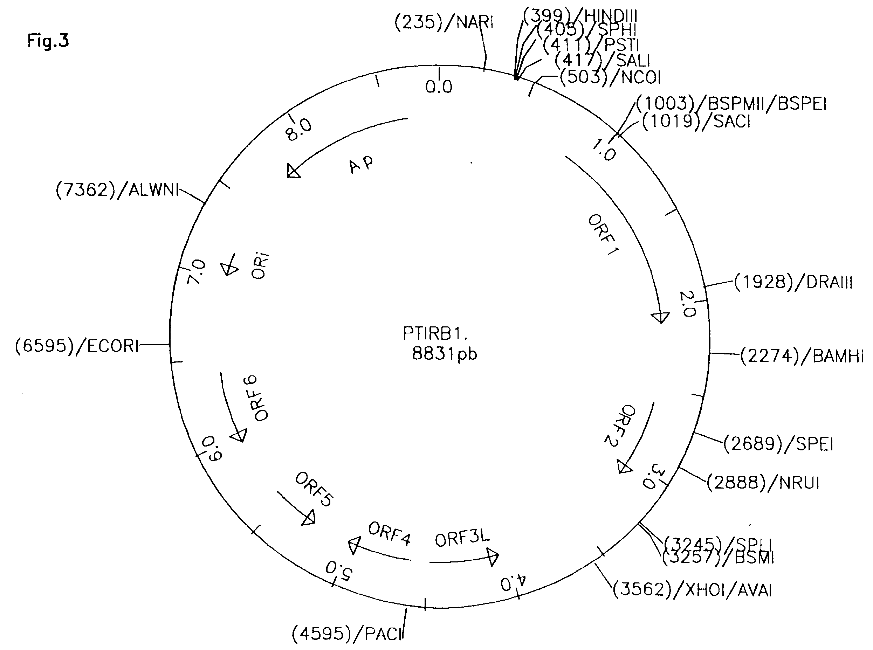

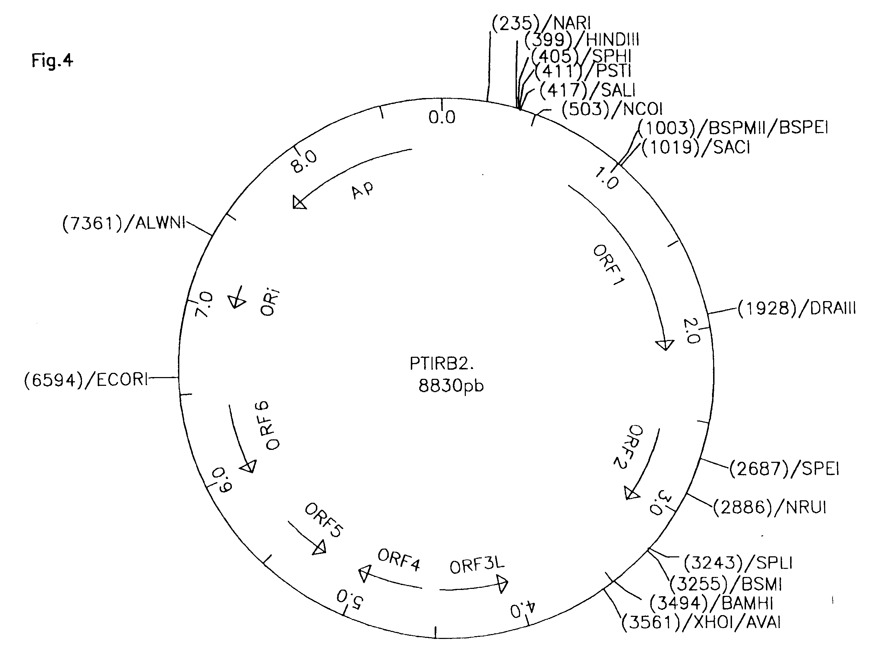

Ces deux ligations ont généré les vecteurs pTIRB1 et pTIRB2. Leurs cartes de restriction sont aux figures 3 et 4.

A partir du plasmide pTIR1, le plasmide pTIRB1D a été généré par délétion de son fragment EcoR1-Hpa1 de 2657 bp.

**Exemple 5 - Clonage des promoteurs P11 et P7.5 de Vaccinia**

P11 et P7.5 sont des promoteurs connus de Vaccinia. P11 est le promoteur du gène codant pour la protéine P11, transcrit au cours de la phase tardive de l'infection virale. P7.5 est le promoteur du gène codant pour la protéine P7.5, transcrit à la fois au cours des phases immédiate et tardive.

Construction de vecteurs avec des sites Bgl2 et Bcl1 uniques

Deux vecteurs de clonage contenant des sites uniques Bgl2, Bcl1 et BamH1 ont été construits pour permettre les clonages successifs de cassettes compatibles avec BamH1. Les séquences des sites de Bgl2 et Bcl1 ont été introduites dans le site BamH1 du pBSPlus par le clonage de l'ADN de synthèse suivant :

```
         Bgl2    Bcl1 BamH1

   5' GATCGAGATCTTGATCAG        3'

   3'     CTCTAGAACTAGTCCTAG  5'
```

Les vecteurs sont pBSLK1 et pBSLK2. Les linkers sont dans l'orientation :

pBSLK1 ... Ava1/Sma1-BamH1-Bcl1-Bgl2-Xba1 ...
pBSLK2 ... Ava1/Sma1-Bgl2-Bcl1-BamH1-Xba1 ...

Clonage du promoteur P7.5

Le fragment Bcl1-BamH1 long de 143 bp du plasmide pGS20 contient la séquence promotrice P7.5 (Mackett et al, 1984). La séquence est présentée ci-dessous (Venkatesan et al, 1981; Cochran et al, 1985). Les promoteurs tardif et immédiat sont soulignés. La dernière base de BamH1 est à 10 bp de l'ATG initiateur du gène.

```
5'TGATCACTAATTCCAAACCCACCCGCTTTTTATAGTAAGTTTTTCACCCATAAATAATAAATA
 Bcl1                                       ----------Tardif
 CAATAATTAATTTCTCGTAAAAGTAGAAAATATATTCTAATTTATTGCACGGTAAGGAAGTAGAa
                                     -------Immédiat
 TCATAAAGAACAGTGACGGATCC
           BamH1
```

Le fragment Bcl1-BamH1 a été cloné dans les sites Bcl1 et BamH1 des plasmides pBSLK1 et pBSLK2 générant respectivement les plasmides p1P75 et p2P75. Pour permettre la digestion Bcl1, pBSLK1 et pBSLK2 sont extraits de la souche JM110 accessible à l'ATCC (American Type Culture Collection) sous la référence ATCC 47013.

Clonage du promoteur P11

La séquence du promoteur P11 a été clonée sous la forme d'un ADN de synthèse à partir des données de séquences de Bertholet et al, 1985. Hanggi et al (1986) ont montré qu'un fragment de 30 nucléotides en amont du premier nucléotide de l'ARNm contient le promoteur.

L'ADN de synthèse est un 40mère portant un site Bgl2 et un site BamH1 à ses extrémités. Cet ADN a été cloné dans pBSLK1, pour générer p1P11 et dans pBSLK2, pour générer p2P11. Le fragment Bgl2-BamH1 de p2P11 a par la suite été cloné dans les sites Xho2 - BamH1 de pACYC184. pACYC184 est un vecteur décrit par Chang et Cohen (1978). La ligation entre Xho2 (GGATCT) et Bgl2 (AGATCT) restaure Bgl2.

La séquence du DNA synthétique portant P11 est :

```
5'GATCAAATTTCATTTTGTTTTTTTCTATGCTATAAATAAG

3'    TTTAAAGTAAAACAAAAAAAGATACGATATTTATTCCTAG

      _____                              _____

      Bgl2                               BamH1
```

## Exemple 6 - Construction d'une cassette portant le gène LacZ compatible avec le site de restriction BamH1

L'expression du gène LacZ permettra le criblage des virus recombinants.

Une cassette Bgl2-BamH1 portant le gène LacZ a été aménagée par mutagénèse dirigée. Cette cassette a été clonée dans le plasmide pBSplus (Stratagène) délété du fragment LacZα et ceci pour éviter la recombinaison entre les deux séquences homologues, l'une sur LacZ l'autre sur LacZα. Ce plasmide est le pBSMutLacZ1. La séquence des extrémités du gène LacZ après mutagénèse est donnée ci-après. Les numéros correspondent aux numéros des acides aminés de la protéine naturelle.

```
En 5',

    Bgl2    Nco1    8    9    10
... AGATCTCACC ATG GCC GTC GTT  ...
               Met Ala Val Val
               <-      β-gal    -

En 3',

... CAA AAA TAA TAA TAA CCGGGCAGG GGGGATCC ...

... Gln Lys *** *** ***
              ->
```

## Exemple 7 - Clonage du gène LacZ derrière les promoteurs P7.5 ou P11

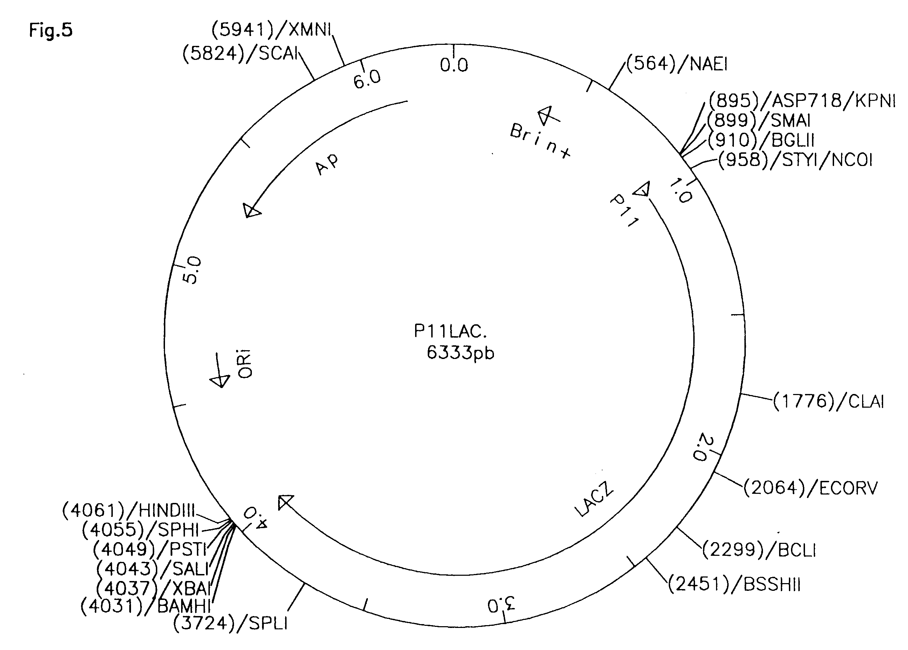

La cassette LacZ, Bgl2-BamH1, du pBSMutLacZ1 a été clonée dans les sites Bgl2-BamH1 de p2P75 et de p2P11 pour générer respectivement p75Lac et p11Lac. On obtient l'expression du gène LacZ chez E. coli lorsqu'il est placé derrière les promoteurs P7.5 ou P11. Les colonies transformées sont de couleur bleue sur un milieu contenant du X-Gal (substrat chromogène : 5-bromo-4-chloro-3-indolyl-β-D-galactopyranoside) qui engendre une coloration bleue sous l'action de la β-galactosidase. Ce test prouve que le gène est fonctionnel. La cassette P11Lac sous la forme d'un fragment Bgl2-Hind3 du plasmide p11Lac a également été clonée dans les sites Xho2 et Hind3 du pACYC184, générant pACP11Lac. La carte de p11Lac est à la figure 5.

## Exemple 8 - Clonage des cassettes P75Lac et P11Lac dans les plasmides pTIRB1 et pTIRB2

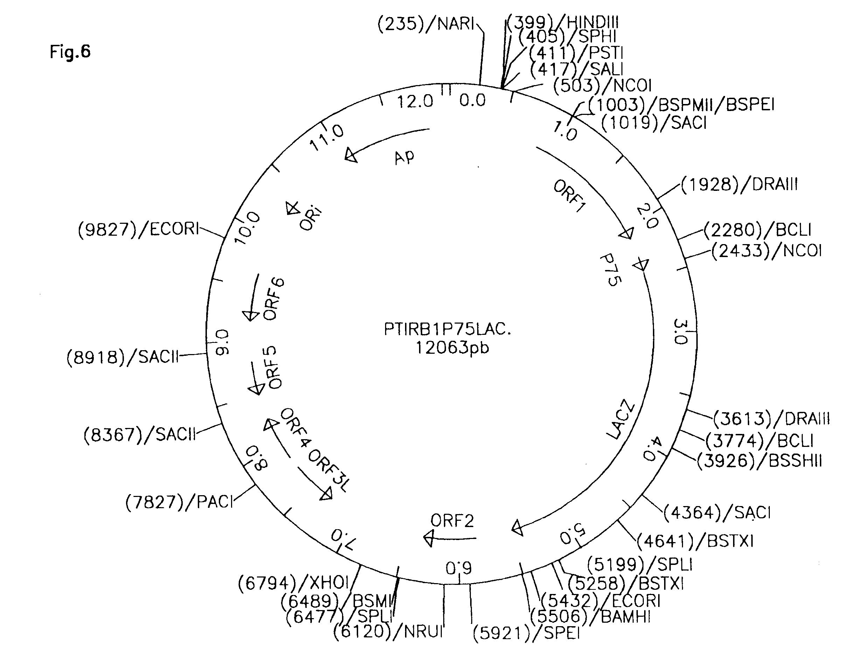

Le fragment Bgl2-BamH1 de p11Lac (ou pACP11Lac) et le fragment Bgl2-BamH1 de p75Lac portent la cassette 'Promoteur P7.5 ou P11 suivi du gène LacZ' (exemple 7). Ces fragments ont été clonés dans les sites BamH1 de pTIRB1 et de pTIRB2 (exemple 4), dans les deux orientations possibles, en nous aidant du phénotype LacZ pour le criblage. Six plasmides ont été isolés parmi les huit recombinants possibles. Leur numérotation est donnée ci-dessous. Convenons que l'orientation '+' est celle qui place la transcription du gène LacZ dans le sens ORF1 vers ORF2 ou ORF2 vers ORF3, et l'orientation '-' celle qui correspond à l'orientation inverse. Dans le nom des plasmides, 'P75Lac' et 'P11Lac' signifient que ces cassettes sont en orientation '+', tandis que 'LacP75' et 'LacP11' signifient que ces cassettes sont en orientation '-'. A titre d'exemple, la carte du plasmide pTIRB1P75Lac est à la figure 6.

| N° | Prom | Site | Ori | Nom | N° | Prom | Site | Ori | Nom |
|----|------|------|-----|-----|----|------|------|-----|-----|
| 3 | P7.5 | B1 | + | pTIRB1P75Lac | 8 | P11 | B1 | + | pTIRB1P11Lac |
| 4 | p7.5 | B2 | + | pTIRB2P75Lac | 9 | P11 | B1 | - | pTIRB1LacP11 |
| 5 | P7.5 | B2 | - | pTIRB2LacP75 | 10 | P11 | B2 | - | pTIRB2LacP11 |

N° = numéro donné au plasmide.
Prom = promoteur.
Site B1 ou B2 = site de clonage BamH1 introduit dans TIR.
Ori = orientation. L'orientation est + lorsque le sens de transcription de LacZ est dans le sens ORF1 vers ORF2 ou ORF2 vers ORF3. L'orientation - est l'inverse.
Nom = nom du plasmide de transfert.

## Exemple 9 - Mise au point des conditions de transfert et de criblage et isolement de virus recombinants CNP/LacZ

Le principe de la construction d'un virus CNP recombinant est présenté à la figure 1. Le protocole de transfert est le suivant :

Au jour J=1, par flasque de 25 cm$^2$, on inocule 2,5 10$^6$ cellules QT35 dans 5 ml de milieu de croissance (composition décrite à l'exemple 1).

Au jour J=2, on effectue le transfert :

Virus : un flacon de 1 ml de vaccin CNP lyophilisé est réhydraté par 3 ml d'eau Milli Q stérile et conservé à -70°C. Ce stock de virus est dégelé et soumis à une sonication douce durant 1 minute. On dilue ensuite dans du milieu de culture référencé E119-F12 et qui correspond au milieu E119 décrit à l'exemple 1 sans LAH, ni sérum, ni fructose pour atteindre une multiplicité d'infection de 0.05 virus par cellule dans 2 ml de milieu. On remplace alors le milieu de culture par la suspension virale et on incube la culture 2 h à 38°C.

Plasmides : on mélange 20 µg de plasmide dans 400 µl d'eau et 100 µl de CaCl$_2$, 1.25 M, on ajoute goutte à goutte 500 µl du tampon BBS (BES Buffer Saline) 2 fois concentré (50 mM de BES (Sigma), 280 mM NaCl, 1.5 mM Na$_2$HPO$_4$; pH 7.0) et on incube entre 15 à 30 minutes à 25°C. Un ml de la préparation de plasmides est ensuite mis sur les cellules, après élimination du milieu. Puis on incube 30 minutes à 38°C. On ajoute alors 4 ml de milieu sans LAH additionné de 5 % de FCS et de 15 mM Hepes (Sigma) pH 7,2. On incube à nouveau 4 heures à 38°C. Ensuite on remplace le milieu par 5 ml de milieu de croissance référencé E199 (composition décrite à l'exemple 1).

Au jour J=0, on récolte les virus comme décrit à l'exemple 1.
Le criblage se base sur la production de β-galactosidase mise en évidence par essai sur boîte de Pétri "plaque assay". Le protocole pour une boîte de Pétri de 6 cm est le suivant :

Au jour J=1 : on inocule $2,5 \times 10^6$ cellules dans 5 ml de milieu de croissance.

Au jour J=2 : on infecte cette culture avec 1 ml de virus aux dilutions 1:10 et 1:100, on incube 4 h à 38°C puis on ajoute 4 ml de milieu de croissance (composition décrite à l'exemple 1).

Au jour J=3 : on remplace le milieu par 5 ml d'une couche d'agarose composée de 1 volume d'agarose 2 % [Sea Plaque Agarose (FMC) dissout dans l'eau] et de 1 volume du mélange suivant : 9 ml de milieu 199 2X (Gibco), 0.5 ml de LAH (Gibco) et 0.1 ml de Hepes (pH 7.2, 15 mM final) et 1.0 ml de FCS. L'agarose fondu et le mélange sont maintenus à 38°C avant d'être mélangés. On laisse gélifier l'agarose 30 minutes à température ambiante puis on incube à 38°C.

Au jour J=6 : on recouvre la culture par 2 ml de 1 % agarose dans du PBS (Gibco) contenant 0.3 mg/ml de X-Gal (5-Bromo 4-Chloro 3-Indolyl β-D-Galactopyranoside, Boehringer). Le X-Gal est dissout à 30 mg/ml dans du DMSO (Dimethyl sulfoxyde, Fluka).

Au jour J=7 : on compte le nombre et la proportion de plages bleues par rapport au nombre de plages non colorées. Puis on prélève individuellement 5 plages à l'aide d'une pipette Pasteur. On conserve les virus dans 500 µl de milieu de croissance à -70°C.

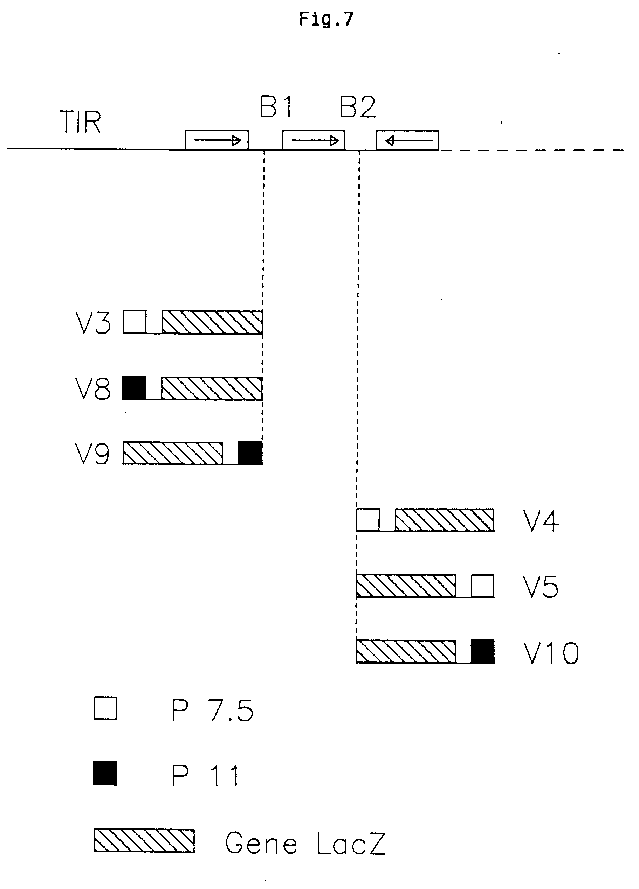

Les fréquences de recombinaison pour chaque plasmide sont reprises dans le tableau ci-dessous. La notation des virus recombinants est 'V' suivi du numéro du plasmide de transfert (tel que défini à l'exemple 8). Le pourcentage de recombinants varie de 0.1 % à 0.5 %, ce qui correspond aux données de la littérature pour les Pox. Une représentation des génomes recombinants est schématisée à la figure 7.

| Virus | Bleu/total | % | Virus | Bleu/total | % |
|-------|-----------|-----|-------|-----------|------|
| V3 | 7/1400 | 0.5 | V8 | 1/1000 | 0.1 |
| V4 | 10/2000 | 0.5 | V9 | 60/25000 | 0.25 |
| V5 | 15/5000 | 0.3 | V10 | 50/30000 | 0.17 |
| Bleu/total = nombre de plages bleues/nombre total de plages<br>% = pourcentage de plages bleues. | | | | | |

## Exemple 10 - Purification des virus recombinants CNP/LacZ

Un clone est obtenu par purification successive d'une plage bleue sur boîte de Pétri (comme décrit à l'exemple 9). En principe, lorsque toutes les plages sont bleues, tous les virus expriment le gène LacZ et ne sont plus contaminés par du virus sauvage. Trois à quatre passages sont nécessaires pour atteindre cette homogénéité. A titre d'exemple, l'évolution de la proportion des plages bleues au cours des passages a été :

| Nombre de passage | | | | | | | | |
|---|---|---|---|---|---|---|---|---|
| | 1er | | 2ème | | 3ème | | 4ème | |
| Virus | Bleu/total | % | Bleu/total | % | Bleu/total | % | Bleu/total | % |
| V3 | 20/200 | 10 | 40/50 | 80 | 22/22 | **100** | | |
| V4 | 1/20 | 20 | 20/30 | 70 | 95/100 | 95 | 100/100 | **100** |
| V5 | 50/500 | 10 | 2/5 | 40 | 30/30 | **100** | | |
| V8 | 50/200 | 25 | 4/5 | 80 | 8/8 | **100** | | |
| V9 | 1/5 | 20 | 1/1 | **100** | | | | |

(suite)

| Nombre de passage | | | | | | | | |
|---|---|---|---|---|---|---|---|---|
| | 1er | | 2ème | | 3ème | | 4ème | |
| Virus | Bleu/total | % | Bleu/total | % | Bleu/total | % | Bleu/total | % |
| V10 | 1/100 | 1 | 3/20 | 15 | 12/12 | **100** | | |
| Bleu/total = nombre de plages bleues/nombre total de plages<br>% = pourcentage de plages bleues. | | | | | | | | |

Sur une photographie en microscopie électronique d'une coupe des cellules QT35 infectées par le virus recombinant V8, des structures typiques des virus Pox sont clairement visibles, en particulier la structure interne qui a la forme d'un os de chien.

## Exemple 11 - Production d'une grande quantité de chaque recombinant

Les six recombinants ont été amplifiés par trois passages successifs sur flasques. Le titre du second passage varie de $4\ 10^5$ à $1.1\ 10^7$ TCID50 (Tissue Culture Infectious Dose) par ml. Seul V10 a un titre plus faible de $10^4$ . Les autres recombinants se multiplient en fait aussi bien que le sauvage pour lequel les titres se situent habituellement entre $10^5$ et $10^7$.

## Exemple 12 - Analyse par "plaque assay" de la stabilité du gène LacZ inséré en TIR

Le gène LacZ inséré dans le génome viral peut être stable ou non, en fonction du type de recombinaison qui a eu lieu (ceci est explicité par Shuman et al, 1989). Ainsi, si il y a deux recombinaisons, de part et d'autre de l'insert, le gène LacZ inséré est stable. Par contre, une simple recombinaison entraîne l'insertion du plasmide de transfert complet, et donc de LacZ. Ce virus donne des plages bleues. Mais le génome viral porte des séquences homologues en orientation directe dont la recombinaison peut entraîner la perte de l'insert et l'apparition de plages non bleues.

D'autre part, chez les Pox, un changement dans la séquence d'une TIR peut être transféré vers l'autre TIR au cours des cycles d'infection et de réplication du virus. Une double recombinaison peut insérer LacZ en TIR-L, la plage de ce virus apparaît bleue. Au cours des infections suivantes, une recombinaison entre cette TIR-L recombinante et une TIR-R sauvage génère une population mixte de virus TIR-L/LacZ, TIR-L/LacZ - TIR-R/LacZ et WT (sauvage). D'autres recombinaisons peuvent par ailleurs générer le dernier type possible qui est TIR-R/LacZ. Par conséquent, une plage bleue peut contenir trois types de virus : des virus qui ont perdu le gène LacZ, des virus qui portent une seule copie du gène LacZ et des virus qui portent deux copies du gène LacZ.

On teste l'homogénéité d'une préparation virale par un "plaque assay" après quelques infections successives en milieu liquide (exemple 9). Les "plaques assay" des 3ème passages des virus V3, V4, V5, V8, V9 et V10 ont fait apparaître les proportions de bleu suivantes :

| Virus | P3 : Bleu/total | % | P6 : Bleu/total | % |
|---|---|---|---|---|
| V3 | 60/70 | 85 | - | |
| V4 | 80/90 | 88 | 70/80 | 88 |
| V5 | 95/100 | 95 | - | |
| V8 | 50/50 | 100 | 40/40 | 100 |
| V9 | 70/70 | 100 | - | |
| V10 | 3/10 | 30 | - | |
| P3 et P6 = 3ème et 6ème passage du virus.<br>Bleu/total = nombre de plages bleues / nombre total de plages.<br>% = pourcentage de plages bleues.<br>- = pas réalisé. | | | | |

En conclusion, les préparations des virus recombinants V8 et V9 sont homogènes. Le recombinant V8 est stable jusqu'au 6ème passage. Par contre, les préparations V3, V4 et V5 ne sont pas homogènes. Elles contiennent une proportion de 5 à 20 % de virus de type sauvage. Cette proportion est plus élevée encore pour V10.

## Exemple 13 - Analyse des génomes des CNP/LacZ par Southern blot

On teste l'homogénéité d'une préparation virale par Southern blot (Maniatis et al, 1982). Les fragments de restriction des génomes des virus recombinants et des virus non recombinants sont distingués sur base de leur taille.
Le protocole de préparation de l'ADN viral est le suivant :

Au jour J=1 : on inocule une flasque de 25 cm$^2$ contenant 5 ml de milieu de croissance avec $2\times10^6$ cellules QT35.
Au jour J=2 : on infecte à une multiplicité d'infection de 0.01.
Au jour J=5 : on détache les cellules à l'aide d'un racloir (Costar). On centrifuge 10 minutes à 6.000 g. Au culot de centrifugation, on ajoute 500 µl de tampon de lyse composé de TrisHCl 10 mM, pH 8.0, EDTA 10 mM, SDS 0.1 %, RNAse 0.1 mg/ml, Protéinase K 0.1 mg/ml. On transfère les cellules dans un tube Eppendorf. Puis on agite la suspension obtenue et on l'incube 1 heure à 50°C. Ensuite on extrait la phase aqueuse 3 fois au phénol chloroforme. On précipite l'ADN par 0.3 M d'acétate de sodium et 2 volumes d'éthanol à -20°C durant 15 minutes. On centrifuge 10 minutes à 18.300 g. On met le culot de centrifugation en suspension dans 500 µl d'eau. De l'ordre de 5 µl de cette suspension d'ADN total sont suffisants pour un Southern.

### Pour le site B1

Les génomes sont digérés EcoR1. La sonde est le plasmide pTIRB1LacP75 digéré par EcoR1. Cette sonde distingue les fragments TIR-L et TIR-R recombinants et parentaux. Les fragments ont les tailles de 9.3 et 6.2 kb pour le virus sauvage, 7.4, 5.1 (doublet) et 4.4 pour V3, 7.4, 4.9 et 4.4 kb pour V8 et enfin, pour V9, 10.4, 7.3 et 2.0 kb (doublet). Les virus V8 et V9 ont acquis deux copies du gène LacZ, une dans chaque TIR, et ne contiennent pas de fragment TIR de type parental. C'est par contre le cas pour le virus V3.
Puisqu'on peut cloner une copie de LacZ dans chaque TIR, on conclut que le site d'insertion B1 est non-essentiel pour le développement du virus et pour sa croissance sur cellules en culture. D'autre part, le gène LacZ ayant une taille de 3 kb environ, l'isolement de ces doubles recombinants prouve qu'on peut insérer 6 kb par génome du virus CNP.

### Pour le site B2

Les Southern montrent la présence de fragments TIR-L et TIR-R recombinants, mais également des fragments parentaux dans les préparations de V4, V5 et V10. Ces trois préparations ne sont donc pas homogènes.

## Exemple 14 - Alternative pour isoler un double recombinant dans le site d'insertion B1

L'évolution des recombinaisons dans les TIR prédit l'apparition de double recombinant stable dans la descendance d'un virus porteur d'une TIR sauvage et d'une TIR recombinante. Des plages virales sont ré-isolées d'un plage bleue à partir d'une boîte de Pétri "plaque assay", soit par un nouveau essai "plaque assay", soit sur des plaques de titration à 96 puits. On peut analyser le génome de chaque nouvelle plage soit par Southern, soit par PCR. Ces différentes possibilités ont été exploitées.
Le Southern effectué sur les génomes des virus de 10 plages bleues différentes issues d'un "plaque assay" du 3$^{ème}$ passage de V3 a montré que, sur les 10 plages, une préparation est homogène et contient une insertion de LacZ dans chacune des TIR.
Pour la plaque de titration, la dilution est telle qu'il y a zéro ou une plage virale par puit. Cette dilution optimale est évaluée par une titration préalable. De bons résultats sont obtenus en infectant une plaque de titration (200 µl par puit, 20 ml par plaque) avec 50 µl de la suspension virale issue d'une boîte de Pétri "plaque assay" reprise dans 1 ml. Lorsque les plages sont bien visibles, les puits qui contiennent une plage sont notés. Après un gel suivi d'un dégel, 100 µl de milieu, par puit, sont prélevés.
Pour le PCR, l'ADN total est rapidement extrait dans les conditions suivantes : on centrifuge 10 minutes à 10 000g, on reprend le culot dans 200 µl de tampon de lyse (Tris-HCl 10mM, pH 8.0, EDTA 10mM, SDS 0.1%), additionné de RNase (0.1 mg/ml) et de protéinase K (0.1 mg/ml) et on incube le mélange ainsi obtenu 1 heure à 50°C. Puis on extrait la phase aqueuse trois fois au phénol/chloroforme. De cette phase aqueuse on précipite l'ADN à l'éthanol. On reprend le culot obtenu dans 50 pl d'eau. On utilise 10 µl de cette suspension par PCR. Les conditions de PCR mises en oeuvre sont celles recommandées par Perkin Elmer (GeneAmp DNA Amplification Reagent Kit). Les fragments amplifiés sont analysés sur gel d'agarose.

Les primers 5168 et 5169 hybrident de part et d'autre du site d'insertion B1, respectivement en aval et en amont du site. Leur séquence est la suivante.

| Numéro | Séquence de 5' vers 3' | Complémentaire à |
|--------|------------------------|------------------|
| 5169 | 5' CCTTTACTGTTAGTTCTACAATCGAAATTATGC 3' | FPV |
| 5168 | 5' CATGTAGATTTTAATCCGTTAACACGCGCAG 3' | FPV |
| 3254 | 5' GCCGGAAAACCTACCGGATTGATGG 3' | LacZ |

Un fragment de 220 bp est amplifié sur les TIR sauvages et un fragment de plus de 3kb pour la TIR recombinante contenant le gène LacZ. Pour la mise en évidence du gène LacZ, une seconde amplification utilise le primer 5168 situé en aval du site B1 et le primer 3254 situé dans le gène LacZ. Un fragment de 674 bp est amplifié. Ce choix judicieux des primers permet donc de distinguer les TIR recombinantes et les TIR non recombinantes. Une suspension virale est considérée homogène, double recombinant, si le fragment WT (sauvage) n'est pas amplifié. Les contrôles sont le génome du virus WT, les plasmides pTIRB1 et pTIRB1Lac.

| Primers | Fragment sauvage | Fragment recombinant |
|---------|------------------|----------------------|
| 5169 + 5168 | 220 bp | 3 kb |
| 3254 + 5168 | -- | 674 bp |
| -- = pas de fragment amplifié. | | |

## Exemple 15 - Expression du gène LacZ en culture de tissus par les virus recombinants CNP/LacZ

L'activité de β-galactosidase (β-gal) est mesurée en utilisant l'o-nitrophenyl- β-D-galactopyranoside (ONPG) comme substrat. L'enzyme convertit l'ONPG en galactose et o-nitrophenol de couleur jaune dont la quantité est mesurée par absorption à 420 nm. Cette absorption est convertie en unité β-gal à l'aide d'une courbe de calibration selon la règle "1 unité βgal = 1 μmol d'ONP produit / $3 \times 10^6$ cellules / 60 minutes d'incubation des extraits à 28°C".

Cet essai "β-Gal" est mené comme suit, pour une flasque de 25 cm$^2$ de surface. Chez les Pox, on distingue les phases immédiate et tardive de l'infection. La phase immédiate est la plus courte; elle dure environ 6 heures. Elle se termine au moment de la réplication du génome. La phase tardive commence à la réplication et se termine à la fin du cycle infectieux par la libération de particules virales, soit trois jours plus tard. Pour avoir une expression suffisante de LacZ au cours de la phase immédiate, on prolonge artificiellement la phase immédiate par de la cytosine β-D arabinofuranoside (AraC) qui est un inhibiteur de la réplication.

a. Cellules

Au jour J=1, par flasque, on inocule $3 \times 10^6$ cellules QT35 dans 5 ml de milieu de croissance.

Au jour J=2 : on infecte à une multiplicité d'infection de 3 virus par cellule. On incube 1 heure à 38°C. Ensuite on enlève l'inoculum viral à l'aide d'une pipette. Puis on ajoute, par flasque, 5 ml de milieu de maintenance additionné ou non d'AraC (Sigma) à 40 μg/ml. On incube les cultures 16 heures à 38°C.

b. Récolte au jour J=3

On racle les cellules de la flasque.

On centrifuge la culture 10 minutes à 6.000 g. On remet le culot de centrifugation en suspension dans 500 μl de PBS, on mélange cette suspension et on la transfère dans un tube Eppendorf de 1.4 ml.

Ensuite on lyse les cellules par l'addition de 50 μl de CHCl$_3$ et 5 μl de SDS 10 %. On mélange la suspension de cellules brièvement. Puis on centrifuge cette suspension 5 minutes à 9.000 g.

c. Réaction enzymatique

Le substrat ONPG pour 25 échantillons est préparé comme suit : 27.7 mg ONPG (Sigma) et 50 ml de diluant composé de 1 ml MgSO4 0.1 M et 1 ml 2-mercaptoéthanol (Merck) dans un volume final de 100 ml de tampon (90 ml NaHPO$_4$ 0.1M pH 7.0, MgSO$_4$ 0.1M, mercaptoéthanol 1 ml).

On mélange 1.95 ml de la solution ONPG et 50 µl de la suspension de cellules, que l'on incube 1 heure à 28°C. Puis on ajoute à cette suspension 2 ml de Na$_2$CO$_3$ 1M et on mesure l'absorbance de cette suspension à 420 nm.

La comparaison de l'activité β-gal pour les virus recombinants est donnée ci-dessous en unité β-gal.

| Virus | Immédiate (AraC) | | Immédiate+Tardive (-AraC) | |
|---|---|---|---|---|
| | Abs | u β-gal | Abs | u β-gal |
| wt | 0 | 0 | 0 | 0 |
| V3 | 0,28 | 57 | 0,58 | 120 |
| V4 | 0,34 | 71 | 0,58 | 120 |
| V5 | 0,26 | 52 | 0,49 | 101 |
| V8 | 0,05 | 10 | 0,65(1:5) | 670 |
| V9 | 0,05 | 10 | 0,31(1:10) | 640 |

wt = de type sauvage, non recombinant.
Immédiate (AraC) = phase de croissance immédiate artificiellement prolongée pendant 16 heures par l'AraC.
Immédiate+Tardive (-AraC) = phases immédiate et tardive naturelles, sans AraC.
Abs = absorbance.
u β-gal = unité de β-galactosidase mesurée.
1:5 et 1:10 = dilutions préalables des extraits 5 fois et 10 fois.

Le promoteur P7.5 de Vaccinia fonctionne au cours des phases immédiate et tardive de l'infection (V3, V4 et V5); le promoteur P11 a une activité tardive uniquement (V8 et V9); le promoteur P11 est plus fort que le promoteur P7.5, comme dans Vaccinia. La régulation temporelle ainsi que la force relative des deux promoteurs sont donc conservées dans le virus CNP, que l'insertion soit en B1 ou B2 et quelle que soit l'orientation de LacZ.

## Exemple 16 - Vaccination de poussins par du virus recombinant CNP/LacZ. Protection contre la variole aviaire. Réponse immunitaire contre la β-gal

On teste le pouvoir vaccinant des recombinants CNP/LacZ. Les deux critères retenus sont la protection contre la variole aviaire et la réponse immunitaire contre la β-galactosidase.

a. Administration par transpercement alaire

Une suspension des virus recombinants V4, V8 ou une suspension de la souche CNP vacciniale ont été injectées à des poussins âgés d'un jour, certifiés dépourvus de pathogènes spécifiques (SPF), par transpercement de la membrane alaire (méthode dite 'wing web')(WW).

Les tests portent sur trois groupes de 28 à 30 poussins, vaccinés par V4 ou par V8 ou par le CNP, et sur un groupe de 15 poussins non-vaccinés comme contrôle négatif.

Chaque poussin vacciné a reçu 10 µl d'une suspension à 5 x 10$^5$ TCID50 (Tissue Culture Infectious Dose) /ml de virus, équivalent à 5x10$^3$ TCID50 par oiseau. A 29 jours, les poussins ont été mis en contact avec du virus virulent (souche Fowl Pox Challenge Virus obtenue de l'Aphis, USA).

L'absence de lésions évaluée dix jours plus tard montre une protection de tous les poussins vaccinés contre la variole aviaire. Par contre la moitié des poussins non-vaccinés présentaient des lésions. L'analyse des titres en anticorps contre la β-galactosidase par un ELISA direct a montré que 21 des 29 (72%) poussins vaccinés par les virus V4 et 20 des 28 (71%) poussins vaccinés par le virus V8 présentent une séroconversion, c'est-à-dire sont séropositifs.

Le titre ELISA étant la dernière dilution qui donne une densité optique supérieure à 100, le titre ELISA anti-β galactosidase moyen dans les sérums de ces poussins est de 1:800.

b. Administration par voie intramusculaire et sous cutanée

Des poussins d'un jour sont vaccinés au premier jour par $10^{4.4}$ TCID50 virus V8 par voie intramusculaire (IM, 27 poussins) ou sous cutanée (SC, 34 poussins). Ils sont soumis à une épreuve 27 jours plus tard. Ils sont protégés contre la variole aviaire (100%). La séroconversion contre la β-galactosidase est observée chez 24 des 27 (88%) poussins vaccinés IM et chez 27 des 34 (79%) des poussins vaccinés SC.

En conclusion, les résultats d'immunisation montrent :

- que les virus recombinants qui contiennent le gène LacZ dans les régions TIR de leur génome, au site B1 ou B2, ont conservé leur pouvoir immunogène;
- que les promoteurs P7.5 et P11 fonctionnent chez l'animal;
- qu'une réponse anticorps est induite contre la β-galactosidase, considérée ici comme une protéine hétérologue exprimée par le recombinant CNP.
- que les voies intamusculaire (IM), sous cutanée (SC) et par transpercement de la membrane alaire (WW) sont équivalentes, à la fois en terme de protection contre la variole aviaire et de pourcentage de séroconversion contre la β-galactosidase.

**Exemple 17 - Insertion des gènes qui codent pour la glycoproteine E2 du virus de la bronchite aviaire dans les vecteurs de transfert**

Le virus responsable de la bronchite infectieuse aviaire (IBV) est un coronavirus. L'antigène de surface le plus important de ce virus est la protéine E2, composée de deux sous-unités S1 et S2 (Cavanagh, 1983, Cavanagh et al, 1988). De nombreux sérotypes existent, dont le Massachusetts, noté M41 et des sérotypes hollandais, notamment D1466 et D274 (Kusters et al, 1987).

On développe un vaccin recombinant CNP qui exprime la protéine E2 d'un IBV en vue d'obtenir un vaccin efficace contre le virus responsable de la bronchite infectieuse aviaire (IBV).

Le gène de la protéine E2 du sérotype M41 a été obtenu du Dr. Kusters de l'Université d'Utrecht (NL), ainsi que des larges fragments des gènes E2 des sérotypes D1466, D207 et D274.

Une cassette BamH1 du gène E2 de M41 a été construite par mutagénèse dirigée. Le gène complet de la souche D1466 et un gène complet hybride D207/D274 ont été construits à partir des fragments, également sur des cassettes compatibles avec BamH1. Ces trois cassettes ont été clonées en aval du promoteur P7.5, générant les trois plasmides p75M41, p75D1466 et p75D207.

Les cassettes P7.5-E2 sont clonées dans le site BamH1 du vecteur de transfert pTIRB1 puis la cassette P11Lac est clonée en aval de E2, dans le site BamH1. Ces plasmides de transfert sont pTIRB1P75M41Lac, pTIRB1P75D1466Lac et pTIRB1P75D207Lac. Les virus recombinants sont construits par transfert et purifiés comme décrits aux exemples 9, 10 et 11. Les génomes sont analysés par Southern blot comme décrit à l'exemple 13. L'expression de l'antigène E2 est mise en évidence par des techniques immunologiques ELISA, Westernblot ou immunofluorescence à l'aide d'anticorps spécifiques. Un stock de virus recombinants est produit. Des volailles sont vaccinées avec une dose de ces recombinants. L'efficacité du vaccin est évaluée après une infection par des virus pathogènes selon les méthodes classiques pour la bronchite infectieuse et par évaluation du taux d'anticorps contre le virus.

**Exemple 18 - Insertion d'un gène d'Eimeria dans les plasmides de transfert - Construction de recombinants CNP/TA4**

Le genre Eimeria comprend des parasites de la volaille responsables de la coccidiose. Des antigènes de surface ont été décrits, en particulier pour les espèces E. tenella, E. necatrix, E. maxima (demande de brevet européen 0 164 176 et 0 231 537).

On développe un vaccin recombinant efficace contre la coccidiose.

L'antigène de E. tenella, noté TA4 ou A4, est une protéine de 25 Kd, composée de deux sous-unités de 17 Kd et 8 Kd reliées par un pont disulfure. Le gène a été isolé d'une banque génomique et également à partir du mARN. La description complète du gène a été publiée dans les demandes de brevet précitées.

Une cassette BamH1 du gène TA4 a été construite par sous-clonage. Le plasmide pTA406 contient la séquence codante de TA4. Le plasmide pTA410 porte le gène TA4 avec une modification dans la séquence protéolytique qui sépare les deux sous-unités. Par mutagénèse dirigée, la séquence native Arg-Arg-Leu a été remplacée par la séquence Arg-Glu-Lys-Arg (décrite par Kieny et al, 1988). Ces deux cassettes BamH1 ont été clonées en aval du promoteur P7.5 du plasmide p1P7.5, dans l'orientation qui place le gène TA4 sous le contrôle de P7.5.

Les cassettes P7.5-TA406 et P7.5-TA410 sont clonées dans le site BamH1 du vecteur de transfert pTIRB1D. Puis la cassette P11-Lac est clonée dans le site BamH1, en aval du gène TA4. Les plasmides de transfert sont pTIRTA406Lac et pTIRTA410Lac.

Les virus recombinants ont été obtenus par transfert comme décrits aux exemples 9, 10, 11. Des plages bleues sont obtenues. Le recombinant porteur du gène TA406 est le V20; le recombinant porteur du gène TA410 est le V21.

Un virus V21 double recombinant, c'est à dire porteur d'une copie de TA410 et de LacZ dans chaque TIR, est purifié sur plaque de titration et son génome analysé par PCR, comme décrit à l'exemple 14. Les primers utilisés et la taille des fragments amplifiés sont repris dans le tableau ci-dessous. Le primer 1871 est complémentaire au gène LacZ.

| Primers | Fragments sauvages | Fragments recombinants |
|---|---|---|
| 5169 + 5168 | 220bp | 4 kb |
| 5169 + 1871 | -- | 1.2 kb |

L'expression de l'antigène TA4 est mise en évidence par des techniques immunologiques ELISA, Western blot ou immunofluorescence à l'aide d'anticorps spécifiques.

Un stock de virus recombinants est produit. Des volailles sont vaccinées avec une dose de ces recombinants. L'efficacité du vaccin est évaluée après une épreuve ("challenge") par _Eimeria_ virulents selon les méthodes classiques pour la coccidiose, dont l'analyse des sérums, la vitesse de la prise de poids et les signes cliniques.

## Exemple 19 - Insertion du gène de la polyprotéine du Gumboro dans les vecteurs de transfert

L'agent responsable de la maladie de Gumboro est un virus de la famille des Birnaviridae. Le virus, appelé IBDV (Infectious Bursal Disease Virus) provoque une maladie fort contagieuse (maladie de Gumboro) qui affecte les jeunes poulets et se caractérise par la destruction des cellules lymphoïdes de la bourse de Fabricius. Le virus possède un génome constitué de 2 segments d'ARN double brin, appelés segment A (environ 3400 paires de bases; pb) et segment B (environ 2900 pb). Les particules virales sont non enveloppées et de forme icosahédrale d'environ 60 nanomètres de diamètre. Quatre protéines virales ont été clairement identifiées : VP1, d'un poids moléculaire (PM) de 90 K (K: kilodalton); VP2, PM de 37 K à 40 K; VP3, PM de 32 K à 35 K et VP4, PM de 24 K à 29 K (Dobos 1979; Fahey et al, 1985). VP2 est dérivée d'un précurseur VPX d'un PM de 41 K à 54 K.

Le segment B code pour VP1 qui est vraisemblablement la polymérase virale. Le segment A code pour les 3 autres protéines. Celles-ci sont générées par clivage protéolytique à partir d'un précurseur d'un PM d'environ 110 K correspondant à une grande phase ouverte de lecture du segment A. La protéine VP4 est impliquée dans ce clivage protéolytique (Jagadish et al 1988). Les protéines VP2 et VP3 constituent la capside virale. VP2 contient les déterminants antigéniques capables d'induire la synthèse d'anticorps neutralisant le virus (Becht et al 1988; Fahey et al 1989).

Un vaccin Fowlpox recombinant contre la maladie de Gumboro est développé.

Les étapes de ce développement sont les suivantes :

1. L'isolement du matériel génétique de la souche IBDV choisie, à savoir la souche EDGAR. La souche de virus EDGAR est accessible auprès du département de l'agriculture des Etats-Unis (USDA, APHIS 6505 Belcrest Road, Hyattsville, MD 20782, Etats-Unis).
2. La synthèse, le clonage et la détermination de la séquence nucléotidique de l'ADNc correspondant au segment A.
3. L'insertion de l'ADNc ainsi que des séquences nécessaires à l'expression de ce matériel génétique dans les cellules animales infectées par le Fowlpox et les séquences permettant le criblage des virus Fowlpox recombinants, dans le plasmide de transfert pTIRB1 décrit dans l'exemple 4.
4. L'isolement, la purification des virus recombinants.
5. L'analyse génétique des virus recombinants.
6. L'analyse de l'expression in vitro, en culture de cellules, des gènes de l'IBDV portés par ces virus recombinants.
7. La vaccination de poussins par les virus recombinants et l'analyse de la protection contre la maladie de Gumboro.

Etape 1

L'ARN viral de la souche EDGAR a été isolé à partir de bourses de poulets infectés par le virus.

Environ 40 g de bourses, recoltées 7 jours après infection par le virus, sont broyées dans 40 ml du tampon TNE (TNE: TRIS-HCl 10 mM, NaCl 100 mM, EDTA 1 mM, pH 8). Le broyat est centrifugé à 17.000 g et la phase aqueuse obtenue déposée sur un gradient de sucrose préformé constitué de 2 couches, 40 % et 60 % en sucrose (% en poids/volume, dans le tampon TNE). Le gradient est centrifugé à 134.000 g durant 2 heures 30 minutes. L'interphase 40 % - 60 % du gradient de sucrose est récoltée et contient du virus partiellement purifié. 5 ml de cette phase sont ajoutés à 5 ml de tampon contenant TRIS-HCl 10 mM, NaCl 100 mM, SDS 0.5 %, EDTA 10 mM, Protéinase K 2 mg/ml, pH 7.5. Le mélange est incubé 1 heure à 37°C. La phase aqueuse est ensuite extraite au phénol/ chloroforme. Les acides nucléiques de la phase aqueuse sont précipités à l'éthanol en présence de 0.8 M de LiCl et repris dans 500 µl d'eau.

Etape 2

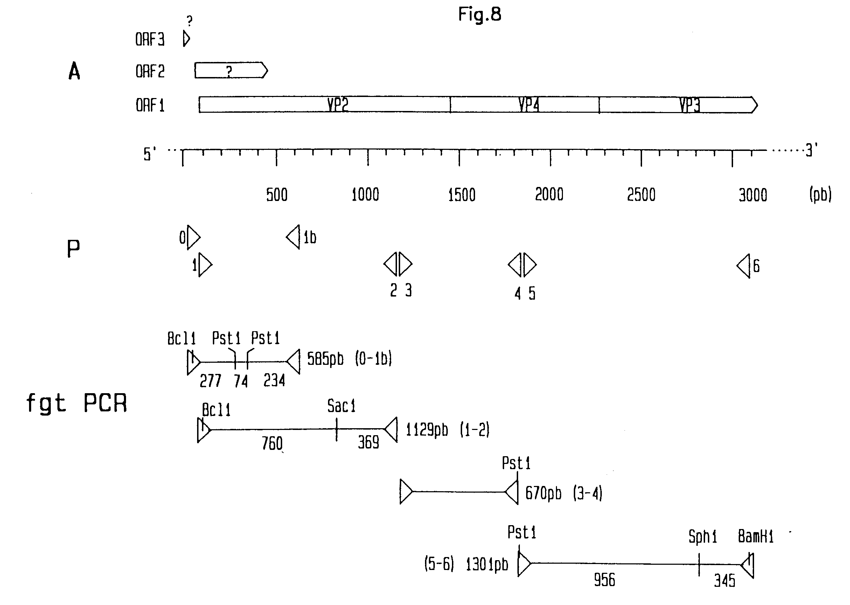

La synthèse et l'amplification de l'ADNc correspondant au segment A ont été réalisées suivant la méthode décrite dans "GENEAMP RNA PCR KIT, PERKIN ELMER CETUS" et à l'aide d'oligonucléotides synthétiques ou "primers", complémentaires à la séquence du segment A, comme amorce de synthèse du premier brin d'ADN par la transcriptase inverse. Le choix de ces oligonucléotides synthétiques a été défini par l'analyse des séquences publiées des segments A d'autres souches d'IBDV: la souche Australienne 002-73 (Hudson et al, 1986), la souche allemande CU-1 (Spies et al, 1989) et la souche Britannique 52/70 (Bayliss et al, 1990). La séquence des primers et leur position par rapport au segment A de la souche EDGAR sont reprises sur les figures 8 et 9.

L'analyse des séquences publiées des souches d'IBDV indique qu'en plus d'une grande phase ouverte de lecture, ORF1, codant pour les protéines VP2, VP4 VP3, il existe deux autres phases ouvertes de lecture sur le même brin codant du segment A. L'une, ORF2. initiée 34 pb en amont de l'ATG de l'ORF1 chevauche cette dernière et a une longueur de 435 pb. L'autre, ORF3, est initiée 32 pb en amont de l'ORF2 et a une longueur de 31 pb. Le rôle éventuel de ces ORF dans la biologie du virus n'est actuellement pas défini.

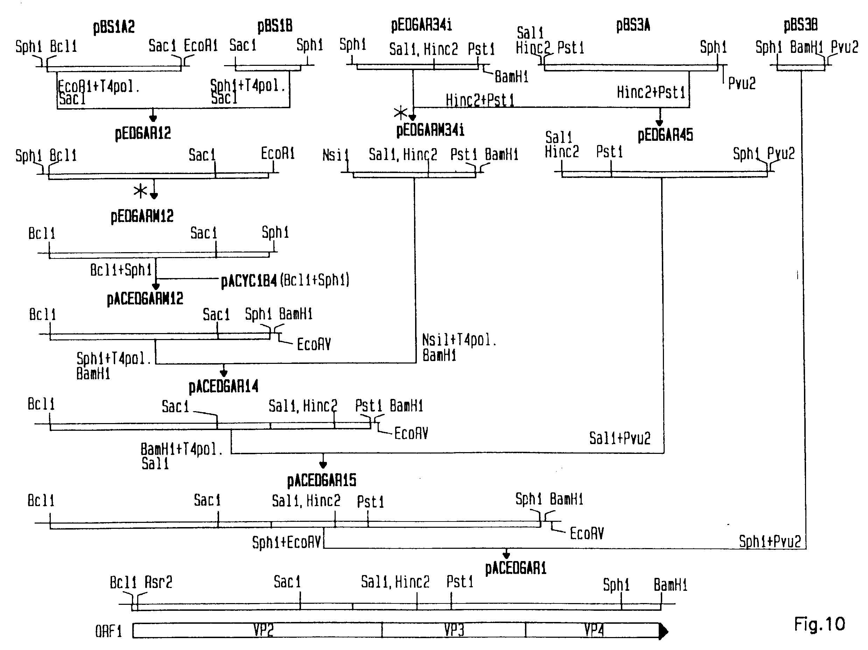

Quatre fragments d'ADNc double brin ont été générés, délimités respectivement par les paires de "primers", 0-1b (585 pb), 1-2 (1129 pb), 3-4 (670 p), 5-6 (1301 pb) et couvrant les ORF1 et ORF2 du segment A (voir figure 8). Ces fragments ont été clonés dans les plasmides pBSPlus ou pBSLK1; six plasmides ont ainsi été construits (voir figures 8 et 10) :

| Nom du plasmide | Taille (pb) | Schéma de construction |
|---|---|---|
| pBSIBDVO | 3478 | pBSPlus (Pst1 + Hinc2) + fragment Pst1 de 277 pb du fragment 0-1b |
| pBS1A2 | 3932 | pBSLK1 (Pst1 + T4 DNA polymérase, Sac1) + fragment Sac1 de 760 pb du fragment 1-2 |
| pBS1B | 3538 | pBSLK1 (Pst1 + T4 DNA polymérase, Sac1) + fragment Sac1 de 369 pb du fragment 1-2 |
| pEDGAR34i | 3887 | pBSLK1 (Pst1 + T4 DNA polymérase, Hinc2) + fragment 3-4 de 670 pb |
| pBS3A | 4175 | pBSLK1 (Pst1 + T4 DNA polymérase, Sph1) + fragment Sph1 de 956 pb du fragment 5-6 |
| pBS3B | 3509 | pBSLK1 (Pst1 + T4 DNA polymérase, Sph1) + fragment Sph1 de 345 pb du fragment 5-6 |

Les séquences nucléotidiques des fragments IBDV de ces plasmides ont été déterminées et alignées de manière à reconstituer la séquence du segment A de la souche EDGAR amplifié par PCR (voir figure 9). Les séquences originales de la souche EDGAR, qui ont été remplacées par l'utilisation des "primers" définis sur base des séquences publiées d'autres souches d'IBDV, ont également été déterminées à partir des produits d'amplification de ces régions à l'aide de "primers" situés à l'extérieur de celles-ci.

La reconstruction du segment A, à partir des clones obtenus, est schématisée sur la figure 10.

Les étapes sont les suivantes :

1. Construction de pEDGAR12 :

pBS1A2 (<u>Eco</u>R1 + T4 DNA polymérase, <u>Sac</u>1)
+ pBS1B (<u>Sph</u>1 + T4 DNA polymérase, <u>Sac</u>1)

2. Construction de pEDGARM12 par mutagénèse dirigée sur pEDGAR12 :

a. délétion du site <u>Sph</u>1 de pEDGAR12 situé en amont de la séquence IBDV :

```
                                            Sph1
Séquence originale      5' GTCTGATCTCTACGCATGCAAGCTTTTGTTCCCTTTAGTGAGGG 3'

"Primer" de mutagénèse 5' GTCTGATCTCTACGGTTCCCTTTAGTGAGGG 3'
```

b. délétion du site <u>Eco</u>R1 de pEDGAR12 et introduction d'un site <u>Sph</u>1, en aval de la séquence IBDV;
séquence originale :

```
        séquence originale :
                            EcoR1
   5' CGACTCACTATAGGGCGAATTCCCCCCAGCGACCGTAACGACTG 3'
                              <-          IBDV      -
        "Primer" de mutagénèse :
                            Sph1
   5' CGACTCACTATAGGGCGGATCCCGCATGCCCCAGCGACCGTAACGACTG 3'
                              <-     IBDV          -
```

3. Construction de pACEDGARM12 :

Insertion du fragment <u>Bcl</u>1-<u>Sph</u>1 de pEDGARM12 dans les sites <u>Bcl</u>1-<u>Sph</u>1 de pACYC184

4. Construction de pEDGARM34i par mutagénèse dirigée sur pEDGAR34i :

Remplacement du site <u>Sph</u>1 par un site <u>Nsi</u>1;

```
              Remplacement du site Sph1 par un site Nsi1;

                                                    Sph1
        Séquence originale :     5' GCTCGAAGTTGCTCAGGCATGCAAGCTTTTG 3'
                                 -    IBDV    ->
                                                    Nsi1
        "Primer" de mutagénèse : 5' GCTCGAAGTTGCTCATGCATGCAAGCTTTTG 3'
                                 -    IBDV    ->
```

5. Construction de pEDGAR45 :

pBS3A (Hinc2 + Pst1) + fragment Hinc2-Pst1 de pEDGAR34i

6. Construction de pACEDGAR14 :

pACEDGARM12 (Sph1 + T4 DNA polymérase, BamH1)
+ pEDGARM34i (Nsi1 + T4 DNA polymérase, BamH1)

7. Construction de pACEDGAR15 :

pACEDGAR14 (BamH1 + T4 DNA polymérase, Sal1) + pEDGAR45 (Pvu2-Sal1)

8. Construction de pACEDGAR1 :

pACEDGAR15 (Sph1 + EcoRv) + pBS3B (Sph1 + Pvu2).

Ce plasmide contient la séquence complète de l'ORF1 du segment A sur le fragment Bcl1-BamH1.

9. Construction de pBSIBDVOb par mutagénèse dirigée de PBSIBDVO :

Introduction de l'ORF3 dans pBSIBDVO, qui porte le début des ORF1 et ORF2;

```
Séquence originale:
       -      pBSplus       ->                                    --ORF2-->
5'  CCCGGGGATCCTCTAGAGTCCCTCCTTCTACAACGTGATCATTGATGG 3'
                                                      Bcl1
```

"Primer" de mutagénèse :

```
                                                                --ORF2-->
       -      pBSplus       ->           --------------ORF3-------------->
5'  CCCGGGGATCCTCTAGAGTCTGATCAGGATGGAACTCCTCCTTCTACAACGCTATCATTGATGG 3'
                        Bcl1
```

10. Construction de pACEDGAR2 et pACEDGAR3 :

Les plasmides pACEDGAR2 et pACEDGAR3 ont été obtenus par échange de la région Bcl1-Rsr2 du plasmide pACEDGAR1 par le fragment Bcl1-Rsr2, respectivement, des plasmides pBSIBDVO et pBSIBDVOb.

Les 3 plasmides pACEDGAR1, pACEDGAR2 et pACEDGAR3 permettent donc d'isoler sur un fragment Bcl1-BamH1, respectivement, l'ORF1, les ORF1-ORF2 et les ORF1-ORF2-ORF3 du segment A de la souche EDGAR.

Les séquences de ces 3 plasmides, du côté de l'ATG des ORF sont représentées ci-après :

```
                     Bcl1
    pACEDGAR1:  5'  TGATCACAGCGATGACA ... 3'
                          ---ORF1---->
                     Bcl1
    pACEDGAR2:  5'  TGATCATTGATGGTTAGTAGAGATCAGACAAACGATCGCAGCGATGACA ... 3'
                          --------------------ORF2--------------------->
                                                           ---ORF1---->


                     Bcl1
    pACEDGAR3:  5'  TGATCAGGATGGAACTCCTCCTTCTACAACGCTATCATTGATGGTTAGT ... 3'
                          --------------ORF3-------------->
                                                   ------ORF2----->
```

Etape 3

Ces fragments Bcl1-BamH1 ont ensuite été clonés dans le site BamH1 du plasmide p2P75, générant les plasmides p75EDGAR1, p75EDGAR2 et p75EDGAR3, et plaçant la ou les séquences codantes du segment A sous le contrôle du promoteur P7.5. Ces séquences codantes ont également été placées sous le contrôle du promoteur P11. Le fragment Bcl1-BamH1 du plasmide pACP11 et portant le promoteur P11 a été inséré dans le site BamH1 des plasmides pACEDGAR1, pACEDGAR2 et pACEDGAR3 pour générer, respectivement, les plasmides p11EDGAR1, p11EDGAR2 et p11EDGAR3.

Des cassettes Bcl1-BamH1 P75-EDGAR ou P11-EDGAR ont été isolées de ces différents plasmides et insérées dans le site BamH1 du plasmide pTIRB1 produisant les plasmides pTIR75EDGAR1, pTIR75EDGAR2, pTIR75EDGAR3, pTIR11EDGAR1, pTIR11EDGAR2 et pTIR11EDGAR3. L'orientation des cassettes Bcl1-BamH1 dans le plasmide pTIRB1 qui a été conservée est telle que le sens de la transcription initiée à partir des promoteurs P7.5 et P11 coïncide avec celui des ORF1 et ORF2 présentes dans le plasmide pTIRB1.

Le fragment Bgl2-BamH1 du plasmide pACP11LAC, portant la cassette P11-LACZ, a été inséré dans le site BamH1 des plasmides pTIR75EDGAR1 et pTIR75EDGAR2 pour générer, respectivement, les plasmides pTIR75E1LAC et pTIR75E2LAC. L'orientation de P11-LACZ dans ces plasmides est telle que le sens de transcription du gène LACZ coïncide avec celui des ORF1 et ORF2.

De manière analogue, les plasmides pTIR11E1LAC, pTIR11E2LAC et pTIR11E3LAC ont été générés par insertion, au site BamH1, respectivement des plasmides pTIR1EDGAR1, pTIR11EDGAR2 et pTIR11EDGAR3, de la cassette P75-LACZ isolée sous forme d'un fragment Bgl2-BamH1 du plasmide p75LAC.

Enfin le plasmide pTIR11VP2LAC a été généré en insérant dans les sites PpuM1 (traité à l'ADN polymérase T4) et Bgl2 du plasmide pTIR11E1LAC le fragment Xho1 (traité à l'ADN polymérase T4)-Bgl2 du plasmide p11EDGAR1. Le plasmide de transfert pTIR11VP2LAC contient, sous le contrôle du promoteur P11, la séquence complète de la protéine VP2 ainsi que la partie amino-terminale de la protéine VP4; de par la construction, sont fusionnés, en phase, les trois derniers acides aminés (Asp, Leu, Glu; partie carboxy-terminale de VP3) et le codon de fin de traduction (TGA) de la polyprotéine. En se référant à la figure 9, cette protéine hétérologue correspond donc à une partie de la polyprotéine comprenant les acides aminés 1 à 493 suivis des acides aminés 1010 à 1012, dans laquelle est incluse la protéine VP2.

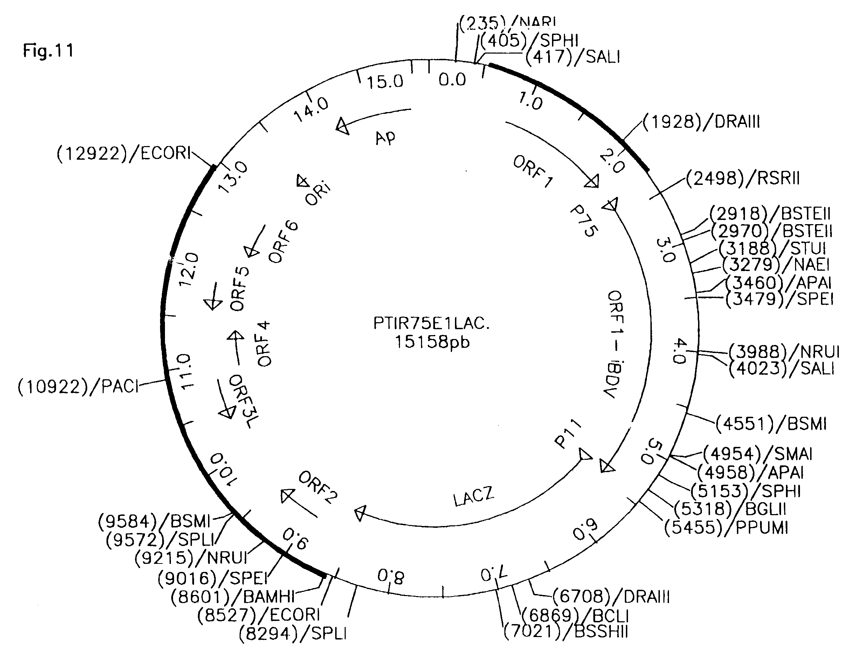

A titre d'exemple, le plasmide pTIR75E1LAC est représenté dans la figure 11.

Etape 4 et Etape 5

Les virus recombinants du Fowlpox dans lesquels ont été intégrés les ORFs de la souche Edgar ont été isolés, purifiés et caractérisés suivant les méthodes décrites dans les exemples 9, 10, 11, 12, 13 et 14.

La notation des virus recombinants est :

| | |
|---|---|
| V11 = P7.5E1 | V12 = P7.5E2 |
| V14 = P11E1 | V15 = P11E2 |
| V17 = P11VP2 | V16 = P11E3 |

Les primers utilisés pour la sélection des TIRs doubles recombinantes en PCR distinguent les TIR WT, les TIR contenant LacZ, E1, E2, E3 ou VP2. Des combinaisons de primers et la taille des fragments amplifiés sont dans le tableau ci-dessous.

Les primers IBDV16, 3, 1b et 22 hybrident avec IBDV. Les primers IBDV16, 1b et 3 hybrident avec la partie codante de VP2. Le primer IBDV22 hybride avec la partie codante de VP3, et donc pas avec le génome du V17. Les primers 5168 et 5169 hybrident avec le FPV. Le primer 1871 hybride avec le gène de la β-galactosidase. La séquence de ces primers est définie dans le tableau "Liste et séquences des primers".

| Primers | Virus | Fragments (nt) |
|---|---|---|
| 5169 + 5168 | WT | 220 |
| | recombinants | 6.0 kb |
| 5169 + 1871 | V8 | 310 |
| | recombinants | 3.6 kb |

(suite)

| Primers | Virus | Fragments (nt) |
|---|---|---|
| 5169 + IBDV16 | V11 | 341 |
| | V14, V17 | 339 |
| | V12 | 373 |
| | V15 | 371 |
| | V16 | 402 |
| 5169 + IBDV1b | V17 | 766 |
| IBDV22 + 1871 | V11, V12 | 379 |
| | V14, V15, V16 | 479 |
| | V17 | -- |
| IBDV3 + 1871 | V14, V15, V16 | 2280 |
| | V17 | 732 |

Etape 6

L'expression des antigènes IBDV des virus recombinants dans les cellules QT35 a été comparée.

Les anticorps utilisés, dressés contre la souche d'IBDV Cu-1, ont été aimablement fournis par le Professeur H. Müller, Instittit für virologie, Justus-Liebig-Universität, Giessen, Allemagne.
Ce sont:

1° un sérum polyclonal (N° B22) de lapin hyperimmunisé contre la souche Cu-1

2° un anticorps monoclonal de souris anti-VP3 (N° I/A10) reconnaissant la protéine VP3 sous forme native et dénaturée

3° un anticorps monoclonal de souris anti-VP2 (N° B1) reconnaissant un épitope conformationnel de la protéine VP2; l'anticorps B1 est un anticorps capable de neutraliser la souche virale Cu-1.

a. Conditions expérimentales de l'infection

Au jour J=1, par flasque de 25 cm$^2$, on inocule 2.5 10$^6$ cellules QT35 dans 5 ml de milieu de croissance (composition décrite à l'exemple 1). On prépare huit flasques.

Au jour J=2, on infecte les cellules à une multiplicité d'infection de 0,01 virus par cellule avec chaque virus recombinant, V11, V12, V14, V15, V16, V17, V8 (contrôle négatif) et IBDV (souche Bursine 2, contrôle positif).

Au jour J=5, on récolte les cellules infectées et le milieu après un gel suivi d'un dégel.

b. Analyse des produits d'expression par ELISA de type sandwich

Le test ELISA est réalisé en utilisant comme premier anticorps l'anticorps polyclonal B22 et comme deuxième anticorps soit le monoclonal anti-VP3 (N° I/A10) soit l'anticorps monoclonal neutralisant anti-VP2 (N° B1). Les courbes ELISA sont présentées aux figures 12A, pour l'anti-VP3, et 12B, pour l'anti-VP2.

Les résultats de ces analyses montrent que:

1 - Figure 12A : la protéine VP3 est exprimée dans tous les recombinants (V11, V12, V14, V15 et V16) qui contiennent au moins la séquence codant pour la polyprotéine (ORF1, protéines VP2-VP4-VP3), mais pas dans le recombinant V17, puisqu'il ne contient que la région codant pour VP2 et la partie aminoterminale de VP4, ni le recombinant V8, puisqu'il ne contient aucune séquence d'IBDV.

Le niveau d'expression de la protéine VP3 est plus faible dans les recombinants V11 et V12 que dans les recombinants V14, V15 et V16, ce qui est vraisemblablement en corrélation avec la force des promoteurs présents dans les différents recombinants: l'activité du promoteur p7.5 (recombinants V11 et V12) étant moins forte que celle du promoteur p11 (recombinants V14, V15, V16) (voir exemple 15).

On n'observe pas de différence de niveau d'expression de VP3 en fonction des phases de lectures du segment A introduites dans les différents recombinants: ORF1 pour les recombinants V11 et V14, ORF1 + ORF2 pour les recombinants V12 et V15 et ORF1 + ORF2 + ORF3 pour le recombinant V16.

2 - Figure 12B : la protéine VP2 est exprimée dans tous les recombinants à l'exception de V8 qui ne contient pas de séquences d'IBDV.

Le niveau d'expression est le plus élevé pour le recombinant V17; dans ce cas, il est proche de celui de la protéine VP2 exprimée dans les cellules infectées par le virus IBDV.

On observe à nouveau une corrélation entre la force des promoteurs présents dans les différents recombinants et le niveau d'expression de la protéine VP2.

Ces résultats montrent, d'une part, que l'épitope de la protéine VP2, reconnu par l'anticorps neutralisant B1 dressé contre la souche Cu-1, est également présent dans la protéine VP2 de la souche américaine Edgar, et d'autre part, que la conformation originale de la protéine VP2 est, dans tous les recombinants, conservée au moins dans cette région.

c. Analyse des produits d'expression par Western blot

L'analyse, par Western blot, des protéines produites par les différents recombinants a été réalisée d'une part, à l'aide du sérum polyclonal (B22) reconnaissant l'ensemble des protéines IBDV et d'autre part, à l'aide de l'anticorps monoclonal anti-VP3 (I/A10).

Les résultats, illustrés par la figure 13 A, montrent que le sérum polyclonal reconnait dans les extraits de cellules infectées par l'IBDV essentiellement 3 protéines correspondant à VP2 (~ 40 kd), VP3 (~ 32 kd) et VP4 (~ 28 kd). Une protéine d'environ 46 kd est également observable et pourrait constituer le précurseur (VPX, ~ 48-49 Kd) de VP2.

Pour tous les recombinants (V11, V12, V14, V15 et V16) qui contiennent au moins la séquence codant pour la polyprotéine (ORF1, VP2-VP3-VP4), des protéines d'un poids moléculaire (PM) coïncidant respectivement avec VP3, VP4 et vraisemblablement VPX sont mises en évidence par le sérum polyclonal anti-IBDV (Figure 13 A). Pour le recombinant V17, une seule protéine dont le poids moléculaire est proche de celui de la protéine de fusion VP2 + région amino-terminale de VP4 (~ 52 kd) est détectée.

L'anticorps monoclonal anti-VP3 (Figure 13 B) reconnait essentiellement deux protéines, VP3 et probablement un produit de dégradation de VP3, dans les cellules infectées par l'IBDV et tous les recombinants exprimant la polyprotéine. Aucune protéine d'IBDV n'est reconnue dans le recombinant V17 exprimant la protéine de fusion VP2-VP4.

L'ensemble de ces résultats indiquent que la phase de lecture codant pour la polyprotéine (ORF1) est traduite dans tous les recombinants indépendamment de la présence des phases de lecture additionnelles ORF2 (recombinants V12, V15) ou ORF2 et ORF3 (recombinant V16). Le clivage de la polyprotéine précurseur est correctement réalisé mais semble incomplet en ce qui concerne VP2; il a été suggéré que la maturation complète du précuseur VPX se réaliserait lors ou après le transport des particules virales IBDV vers la surface des cellules (Müller et al, 1982).

Etape 7

On teste le pouvoir vaccinant des recombinants FPV/IBDV.

a. Séroconversion par V11

Une première campagne de vaccination par un recombinant FPV/IBDV a comparé la séroconversion contre la β-galactosidase et contre le virus IBDV pour trois voies d'administration différentes : par voie intramusculaire (IM), sous cutanée (SC) et par transpercement de l'aile (WW ou wing web).

Des poussins d'un jour sont vaccinés avec $10^{4.4}$ virus recombinants V11 selon les trois voies d'administration IM, SC ou WW. Ils sont euthanasiés 11 jours plus tard.

L'analyse des sérums par ELISA direct a montré que 2/14 (14%) poussins vaccinés par voie intramusculaire, 3/6 (50%) poussins vaccinés par voie sous-cutanée et 8/15 (53%) poussins vaccinés par transpercement de l'aile ont des anticorps contre la β-galactosidase, tandis que 5/14 (36%) poussins vaccinés par voie intramusculaire, 1/6 (17%) poussins vaccinés par voie sous cutanée et 1/15 (7%) poussins vaccinés par transpercement de l'aile ont des anticorps contre le virus IBDV.

En conclusion, les protéines de l'IBDV sont exprimées par le recombinant FPV/IBDV11 chez l'animal vacciné et induisent une réponse anticorps chez quelques poussins. Des trois voies d'administration, la voie intramusculaire induit une réponse supérieure aux deux autres.

b. Protection précoce par V11, V15 et V16

Des poussins d'un jour ont reçu une dose de $10^{4.4}$ virus V8 (contrôle négatif), V11, V15 ou V16 par voie intramusculaire et ont été challengés 10 jours plus tard par 100 LD50 (100 fois la dose léthale) du virus 849VB administré par voie oculaire. La souche 849VB est décrite par Van Den Berg et al, 1991.

Les poussins sont euthanasiés 20 jours plus tard.

Le rapport, multiplié par 100, entre le poids de la bourse et le poids total est un indice de protection.

Chez le groupe contrôle vacciné par V8 et non soumis à l'épreuve IBDV, ce rapport est de 0.57 (moyenne de 10 poussins).

Chez le groupe V8 soumis à l'épreuve, ce rapport passe à 0.11 (9 poussins).

Chez les poussins vaccinés par V11, V15 et V16, ces rapports sont respectivement de 0.11 (20 poussins), 0.12 (18 poussins) et 0.12 (18 poussins).

Sur base de ce critère, il n'y a pas de protection précoce contre le virus IBDV par les recombinants V11, V15 et V16.

c. Protection tardive par V11, V15 et V16

Des poussins vaccinés par V8, V11, V15 et V16, comme décrit au point précédent, ont été soumis à l'épreuve IBDV 42 jours après l'injection.

Les sérums prélevés avant l'épreuve sont analysés par la méthode ELISA pour évaluer la réponse anticorps contre la β-galactosidase et le virus IBDV.

La réponse anti-β-galactosidase montre que 100% de séroconversion est obtenue chez tous les poussins vaccinés, soit 70 poussins au total. Les titres varient de 1:200 à 1:51200. Il n'y a pas de différence significative entre les titres obtenus pour V11, dont le gène LacZ est sous le contrôle de P7.5, et V8, V15 et V16, dont les gènes LacZ sont sous le contrôle du promoteur P11.

La réponse anti-IBDV chez les 10 poussins vaccinés par V8 est nulle; elle est positive chez tous les poussins vaccinés par V11, V15 et V16. En détail, les titres des réponses chez les 20 poussins vaccinés par V11 varient de 1:800 à 1:51200, avec une moyenne de 1:6400. Ils sont de 1:6400 à 1:102400, avec une moyenne de 1:25600 pour les 20 poussins vaccinés par V15. Ils sont entre 1:6400 et 1:204800, avec une moyenne de 1:25600 chez les 20 poussins vaccinés par V16. Les taux en anticorps sont nettement plus élevés pour les groupes vaccinés par V15 ou V16 que V11.

Les titres de séroneutralisation de la souche vaccinale PBG68 adaptée à des cellules en culture sont de 1:10 pour V11, comme le contrôle négatif; 4 sérums sur 20 pour V15 et 1 sérum sur 20 pour V16 ont des titres de séroneutralisation de 1:20. Ce titre est donc faible et est observé chez quelques animaux seulement.

Le rapport (poids des bourses / poids total) x100 est en moyenne de 0.66 dans le groupe contrôle V8 et non challengé (10 poussins). Il passe à 0.1 dans le groupe V8 challengé (1 survivant). Il est de 0.1, 0.11, 0.11 pour les trois groupes V11 (3 poussins), V15 (11 poussins) et V16 (8 poussins) respectivement. Il n'y a pas de protection contre l'atrophie des bourses.

La mortalité dans les quatre jours qui ont suivi le challenge a été presque totale pour le groupe contrôle (9 poussins sur 10). Chez le groupe V11, 3 poussins sur 20 ont survécu, tandis que 11 poussins sur 20 vaccinés par V15 et 8 poussins sur 20 vaccinés par V16 ont survécu. On observe donc une protection de l'ordre de 50% des animaux vaccinés par V15 ou V16.

d. Protection par V14, V15 et V17

Quatre groupes de volailles âgées de trois semaines ont reçu 0,2 ml de virus recombinants V8 (contrôle négatif), V14, V15 ou V17. La quantité de virus par poulet était de $10^{6.4}$ TCID50. La voie d'administration est intramusculaire.

Vingt-et-un jours plus tard, du serum est prélevé de chaque animal pour évaluer le titre en anticorps anti-IBDV. Cent fois la dose léthale d'IBDV est injectée aux animaux des groupes V14, V15 et V17 et une partie des animaux du groupe V8 (c'est-à-dire le groupe de volailles ayant reçu le virus recombinant V8).

11 jours plus pard, soit 32 jours après la vaccination, las animaux témoins du groupe V8 et ceux qui ont survécu à l'épreuve, sont euthanasiés et le rapport (poids des bourses / poids des volailles) x100 est déterminé.

Les résultats :

- Mortalité : le nombre de volailles ayant succombé au challenge se repartit comme suit :
   pour V8 : 12 volailles/14 (85%), pour V14 : 21/24 (87 %), pour V15 : 17/25 (68%) et pour V17 : 0/25 (0%).
   En d'autres termes, toutes les volailles vaccinées par le recombinant V17 ont résisté au challenge. Dans ce groupe, la protection est totale. En outre, aucune volaille n'a montré le moindre signe clinique de maladie. La protection de 32% obtenue avec le recombinant V15 est proche de la protection de 50% obtenue dans l'expérience précédente.

- ELISA : les titres moyens évalués contre le virus sont :
  pour V8 titres inférieurs à 1:100 et considérés comme négatifs; pour V14 titres de 1:12800 (de 1:6400 à 1:51200), pour V15 titres de 1:6400 (de 1:800 à 1:25600), pour V17 titres inférieurs à 1:100 sauf pour 3 volailles (1:800, 1:6400 et 1:51200).

  Donc, la réponse en anticorps contre l'IBDV induite par l'expression de la polyprotéine du FPV/IBDV recombinant est très élevée. La réponse contre le virus complet induite par V17 est par contre faible.

- Séroneutralisation : les titres de séroneutralisation contre la souche PBG68 sont en moyenne : pour V8, V14 et V15 titres inférieurs à 1:10 et considérés comme négatifs; pour V17, 5 volailles sur 20 ont un titre supérieur à 1:20, soit, en détail, de 1:20 (deux fois), 1:40, 1:80 et 1:320.

  Donc, une séroneutralisation est observée uniquement dans des sérums des volailles vaccinées par V17.

- Bourses : la moyenne du rapport (poids des bourses/poids total) x100 se présente comme suit : pour V8 non challengés rapport de 0,68 (± 0,13), pour V8 challengés rapport de 0,09 (± 0,01), pour V14 rapport de 0,10 (± 0,02), pour V15 rapport de 0,13 (± 0,04) et pour V17 rapport de 0,34 (± 0,23).

  Donc, une protection partielle des bourses de Fabricius est observée uniquement dans le groupe V17. En fait, 8 volailles sur 24 ont un rapport semblable à celui des témoins non challengés. Les seize autres ont un rapport plus élevé que les témoins challengés.

Conclusions

Les conclusions majeures de ces campagnes de vaccination sont :

- Les recombinants qui expriment la polyprotéine de l'IBDV induisent chez les volailles une forte réponse ELISA, avec des titres contre le virus qui sont élevés.
- Le recombinant qui exprime la portion VP2 de la polyprotéine protège totalement les volailles contre la mortalité et partiellement contre l'atrophie de la bourse.

Liste des plasmides

| Nom | Caractéristiques |
|---|---|

**Vecteurs de clonage dans E. coli**

| | |
|---|---|
| pUC18 | Vecteur de clonage (Messing 1983) |
| pBSPlus | Vecteur de clonage (Stratagene) |
| pBSLK1 | pBSPlus avec un site Bcl1 et Bgl2 dans le poly-linker |
| pBSLK2 | idem pBSLK1 mais les sites Bcl1 et Bgl2 sont dans l'ordre inverse |
| pACYC184 | Vecteur de clonage (Chang, 1978) |

**Promoteurs de Vaccinia**

| | |
|---|---|
| pGS20 | Vecteur de transfert pour Vaccinia (Mackett 1984) |
| p1P75 | pBSLK1 + fragment Bcl1-BamH1 du pGS20 avec le promoteur P7.5 de Vaccinia dans Bcl1-BamH1 |
| p2P75 | idem P1P75 mais dans pBSLK2 |
| p1P11 | pBSLK1 + ADN de synthèse de 40 mères avec le promoteur P11 de Vaccinia dans Bgl2-BamH1 |
| p2P11 | idem p1P11 mais dans pBSLK2 |
| pACP11 | idem p1P11 mais dans Xho2-BamHI de pACYC184 |

**Gène LacZ**

| | |
|---|---|
| pBSMutLacZ1 | pBSPlus avec LacZ sur une cassette Bgl2-BamH1 |
| p75lac | p2P75 + cassette Bgl2-BamH1 avec LacZ dans Bgl2-BamH1 |
| p11Lac | idem p75Lac mais dans p2P11 |
| pACP11Lac | pACYC184 + cassette P11-Lac sur un fragment Bgl2-Hind3 de p11Lac dans Xho2-Hind3 |

**Vecteurs de transfert pour le CNP**

| | |
|---|---|
| pTIR1 | pUC18 avec le fragment EcoRI TIR-L du CNP. Le fragment qui sépare les sites BamH1 du linker de pUC18 du BamH1 du CNP a été délété. |
| pTIRB1 | Insertion d'un BamH1 dans pTIR1, entre ORF1 et ORF2. |

| pTIRB2 | idem pTIRB1 mais entre ORF2 et ORF3. |
| pTIRB1D | idem pTIRB1, mais délétion du fragment EcoR1-Hpa1. |

### Vecteurs de transfert avec LacZ

| pTIRB1P75Lac | pTIRB1 + cassette P75-Lac du p75Lac sur un fragment Bgl2-BamH1 cloné dans BamH1. |
| pTIRB2P75Lac | idem mais dans pTIRB2 |
| pTIRB2LacP75 | idem mais en orientation opposée |
| pTIRB1P11Lac | pTIRB1 + cassette P11-Lac du p11Lac sur un fragment Bgl2-BamH1 cloné dans BamH1 |
| pTIRB1LacP11 | idem mais en orientation opposée |
| pTIRB2LacP11 | idem mais dans le pTIRB2 |

### Plasmides porteurs de E2 d'IBV

| p75M41 | Gène E2 du sérotype M41 cloné en aval de P7.5 |
| p75D1466 | idem mais sérotype D1466 |
| p75D207 | idem mais sérotype D207/D274 |

### Vecteurs de transfert avec E2 d'IBV

| pTIRB1P75M41Lac | Cassettes P7.5-E2 de M41 et P11-Lac clonées dans le site BamH1 de TIRB1. |
| pTIRB1P75D1466Lac | idem mais E2 de D1466. |
| pTIRB1P75D207Lac | idem mais E2 de D207/D274. |

### Plasmides porteurs de TA4 d'Eimeria

| pTA406 | ADNc de l'antigène TA4 d'Eimeria tenella cloné sur une cassette BamH1 |
| pTA410 | idem mais le site protéolytique est différent |
| p75TA406 | gène TA406 cloné en aval de P7.5 |
| p75TA410 | idem mais gène TA410 |
| pTIRTA406 | cassette P7.5-TA406 clonée dans BamH1 de pTIRB1D |
| pTIRTA410 | idem mais cassette P7.5-TA410 |

### Vecteurs de transfert avec TA4 d'Eimeria

| pTIRTA406Lac | Cassette P11Lac clonée dans pTIRTA406 |
| pTIRTA410Lac | idem mais dans pTIRTA410 |

Plasmides pour le clonage de l'IBDV

| | |
|---|---|
| pBSIBDV0 | pBSPlus (Pst1-Hinc2) ce travail + fragment Pst1 de 277 pb du fragment EDGAR 0-1b obtenu par PCR. |
| pBSIBDV0b | dérivé de pBSIBDV0 par mutagénèse dirigée |
| pBS1A2 | pBSLK1 (Pst1 + T4 DNA polymérase, Sac1) + fragment Sac1 de 760 pb du fragment EDGAR 1-2 obtenu par PCR. |
| pBS1B | pBSLK1 (Pst1 + T4 DNA polymérase, Sac1) + fragment Sac1 de 369 pb du fragment EDGAR 1-2 obtenu par PCR. |
| pEDGAR34i | pBSLK1 (Pst1 + T4 DNA polymérase, Hinc2) + fragment EDGAR 3-4 de 670 pb obtenu par PCR. |
| pEDGARM34i | dérivé de pEDGAR34i par mutagénèse dirigée |
| pBS3A | pBSLK1 (Pst1 + T4 DNA polymérase, Sph1) + fragment Sph1 de 956 pb du fragment EDGAR 5-6 obtenu par PCR. |
| pBS3B | pBSLK1 (Pst1 + T4 DNA polymérase, Sph1) + fragment Sph1 de 345 pb du fragment EDGAR 5-6 obtenu par PCR. |
| pEDGAR12 | pBS1A2 (EcoR1 + T4 DNA polymérase, Sac1) + fragment pBS1B (Sph1 + T4 DNA polymérase, Sac1) |
| pEDGARM12 | dérivé de pEDGAR12 par mutagénèse dirigée |
| pACEDGARM12 | insertion du fragment Bcl1-Sph1 de pEDGARM12 dans les sites Bcl1-Sph1 de pACYC184 |
| pEDGAR45 | pBS3A (Hinc2-Pst1) + fragment Hinc2-Pst1 de pEDGAR34i |
| pACEDGAR14 | pACEDGARM12 (Sph1 + T4 DNA polymérase, BamH1) + fragment pEDGARM34i (Nsi1 + T4 DNA polymérase, BamH1) |
| pACEDGAR15 | pACEDGAR14 (BamH1 + T4 DNA polymérase, Sal1) + fragment pEDGAR45 (Pvu2-Sal1) |
| pACEDGAR | pACEDGAR15 (Sph1-EcoRV) + fragment pBS3B (Sph1-Pvu2) |
| pACEDGAR2 | échange du fragment Bcl1-Rsr2 de pACEDGAR1 par le fragment Bcl1-Rsr2 de pBSIBDV0 |

pACEDGAR3          idem avec le fragment Bcl1-Rsr2 de pBSIBDVOb

## Plasmides avec IBDV et un promoteur Vaccinia

p75EDGAR1          fragment Bcl1-BamH1 de pACEDGAR1 inséré dans le site BamH1 de p2P75

p75EDGAR2          idem avec le fragment Bcl1-BamH1 de pACEDGAR2

p75EDGAR3          idem avec le fragment Bcl1-BamH1 de pACEDGAR3

p11EDGAR1          fragment Bcl1-BamH1 portant P11 de pACP11 inséré dans le site BamH1 de pACEDGAR1

p11EDGAR2          idem dans le plasmide pACEDGAR2

p11EDGAR3          idem dans le plasmide pACEDGAR3

## Vecteurs de transfert avec IBDV seul

pTIR75EDGAR1       fragment Bcl1-BamH1 de p75EDGAR1 inséré dans le site BamH1 de pTIRB1

pTIR75EDGAR2       idem avec le plasmide p75EDGAR2

pTIR75EDGAR3       idem avec le plasmide p75EDGAR3

pTIR11EDGAR1       idem avec le plasmide p11EDGAR1

pTIR11EDGAR2       idem avec le plasmide p11EDGAR2

pTIR11EDGAR3       idem avec le plasmide p11EDGAR3

## Vecteurs de transfert avec IBDV et Lac

pTIR75E1LAC        fragment Bgl2-BamH1 de pACP11LAC inséré dans le site BamH1 de pTIR75EDGAR1

pTIR75E2LAC        idem avec le plasmide pTIR75EDGAR2

pTIR11E1LAC        fragment Bgl2-BamH1 de p75LAC inséré dans le site BamH1 de pTIR11EDGAR1

pTIR11E2LAC        idem avec le plasmide pTIR11EDGAR2

pTIR11E3LAC        idem avec le plasmide pTIR11EDGAR3

pTIR11VP2LAC       fragment Xho1 (DNA polymérase T4)-Bgl2 de p11EDGAR1 cloné dans les sites PpuM1 (DNA polymérase T4)-Bgl2 de pTIR11E1LAC

REFERENCES

- Bayliss C.D., Spies U., Shaw K., Peters R.W., Papageorgiou A., Müller H. and Boursnell M.E.G., J. gen. Virology 71, 1303-1312 (1990).
- Becht H., Müller H. and Müller H.K., J. gen. Virology 69, 631-640 (1988).
- Bertholet C., R. Drillien, R. Wittek. 1985. Proc. Natl. Acad. Sci. USA, **82**:2096-2100.
- Campbell J.A., M.M. Binns, F.M. Tomley, M.E.G. Boursnell. 1989. J. Gen. Virol., **70**:145-154.
- Cavanagh, D. 1983. J. Gen. Virol., 64:2577-2583.
- Cavanagh, D. et al. 1988. Virus research, 11:141-150.
- Chang, A.C.Y. et Cohen, S.N. 1978. J. Bacteriol. 134:1141-1156.
- Cho B.R., Avian Diseases vol. 25, n° 4, 839-846 (1981).
- Cochran et al, 1985. J. of Virol., 54 (1), 30-37.
- Coupar et al. 1990. Virology, 179:159-167.
- Dobos P., Journal of Virology 32, 1046-1050 (1979).
- Fahey K.J., O'Donnell I.J. and Azad A.A., J. gen. Virology 66, 1479-1488 (1985).
- Fahey K.J., Erny K. and Crooks J., J. gen. Virology 70, 1473-1481 (1989).
- Hanggi et al, 1986. EMBO Journal, 5 (5), 1071-1076.
- Hudson P.J., McKern N.M., Power B.E. and Azad A.A., Nucleic acids research 14, 5001-5012 (1986).
- Jagadish M.N., Staton V.J., Hudson P.J. and Azad A.A., J. Virology 62, 1084-1087 (1988).
- Kieny et al, 1988. Protein engineering, 2 (3):219-225.
- Kusters, J.G. et al. 1987. J. Gen. Virol. 68:343-352.
- Mackett et al, 1984. J. of virol., 49 (3), 857-864.
- Mackett et al, 1985. DNA cloning. A practical approach, Vol. 2, 191-211.
- Maniatis, T., E.F. Fritisch, and J. Sambrook. 1982. Molecular cloning : a laboratory manual. Cold Spring Harbor Laboratory, Cold Spring Harbor, N.Y.
- Messing, J. 1983. Methods Enzymol. 101:20-78.
- Moss, B. 1990. Annu. Rev. Biochem. 59:661-88.
- Müller H. and Becht H., 1982. Journal of Virology 44 (1), 384-392
- Spehner et al, 1990. J. of Virol., 64:527-533.
- Spies U., Müller H. and Becht H., Nucleic acids research 17, 7982 (1989).
- Shuman et al, 1989. Virology, 170:302-306.
- Tomley et al, 1988. J. Gen. Virol., 69, 1025-1040.
- Venkatesan et al, 1981. Cell, 125, 805-813.
- Van Den Berg, Gonze et Meulemans, 1991. Avian Pathology, 20, 133-143

| Liste des organismes | |
|---|---|
| Nom | Caractéristiques |
| **1. BACTERIES E. COLI** | |
| MC1061 | araD139, (ara, leu)7697, lacX74, galU, galK, hsdR, strA         (Pharmacia) |
| JM1110 | utilisée pour son phénotype dam-. (N3837dam4) |
| **2. VIRUS CNP** | |
| LVCO | souche de challenge Fowlpox standard de l'USDA. |
| CNP | souche vaccinale de Fowlpox (SOLVAY) |
| V3 | virus Recombinant CNP avec la cassette P7.5-LacZ en TIRB1 |
| V4 | idem V3, mais la cassette est en TIRB2 |
| V5 | idem V4, mais la cassette est en orientation opposée |
| V8 | idem V3, mais la cassette est P11-Lac |
| V9 | idem V8, mais la cassette est en orientation opposée |
| V10 | idem V9, mais la cassette est en TIRB2 |
| **3. VIRUS IBDV** | |
| EDGAR | souche EDGAR isolat américain de S.A. EDGAR, Auburn University, propagé 3 fois sur poulet et une fois sur oeufs embryonnés. Standard de challenge de l'USDA. |
| 849VB | souche pathogène, décrite par Van Den Berg et al, 1991. |
| PBG98 | souche adaptée aux cellules CEF. |
| Bursine2 | souche vaccinale adaptée aux cellules QT35. |
| **4. CELLULES** | |
| QT35 | lignée cellulaire de caille. |
| CEF | chicken embryo fibroblasts |
| **5. VIRUS CNP/IBD** | |
| V11 | porte les cassettes P11Lac et P7.5E1 |
| V12 | porte les cassettes P11Lac et P7.5E2 |
| V14 | porte les cassettes P7.5E1 et P11Lac |
| V15 | porte les cassettes P7.5E2 et P11Lac |
| V16 | porte les cassettes P7.5E3 et P11Lac |
| V17 | porte les cassettes P7.5VP2 et P11Lac |
| **6. VIRUS CNP/TA4** | |
| V21 | porte les cassettes P11Lac et P7.57A410 |

| LISTE ET SÉQUENCES DES PRIMERS | | |
|---|---|---|
| Numéro | Séquence de 5, vers 3' | Complémentaire à |
| 5169 | 5' CCTTTACTGTTAGTTCTACAATCGAAATTATGC 3' | FPV |
| 5168 | 5' CATGTAGATTTTAATCCGTTAACACGCGCAG 3' | FPV |
| 3254 | 5' GCCGGAAAACCTACCGGATTGATGG 3' | Lac |
| 1871 | 5' GAAACCAGGCAAAGCGCC 3' | Lac |
| IBDV16 | 5' CCAGGGTGTCGTCCGGAATGG 3' | IBDV |
| IBDV1b | 5' CCCAAGATCATATGATGTGGGTAAGCTGAGG 3' | IBDV |
| IBDV3 | 5' TGAGCAACTTCGAGCTGATCCCAAATCCTG 3' | IBDV |
| IBDV22 | 5' CTGCTCTTGACTGCGATGGAG 3' | IBDV |

**Revendications**

1. Virus de l'Avipox recombinant dérivé d'une souche atténuée de virus d'Avipox et comportant, dans une partie non essentielle de son génome, au moins une séquence d'ADN codant pour tout ou partie d'une protéine hétérologue au virus de l'Avipox, ainsi que les éléments susceptibles d'assurer l'expression de cette protéine dans une cellule infectée par ledit virus recombinant, caractérisé en ce que la partie non-essentielle du génome est constituée par une région intergénique non codante, ayant une séquence de plus de 60 nucléotides et située entre deux phases ouvertes de lecture (ORF), leurs signaux d'expression y compris, cette région intergénique étant choisie parmi la région, dite région β1, située entre les ORF1 et ORF2 de la Région TIR (Répétitions terminales inverses) et la région, dite région β2, située entre les ORF2 et ORF3. de la Région TIR

2. Virus selon la revendication 1, caractérisé en ce qu'il est dérivé d'une souche atténuée de virus de Fowlpox.

3. Virus selon la revendication 1 ou 2, caractérisé en ce qu'au moins une séquence d'ADN est clonée dans les deux régions TIR du virus.

4. Virus selon la revendication 3, caractérisé en ce qu'une séquence d'ADN est clonée dans la région β1 dans chacune des deux TIR.

5. Virus selon l'une quelconque des revendications 1 à 4, caractérisé en ce que la protéine hétérologue est choisie parmi les antigènes du virus de la maladie de Gumboro (IBDV), du virus de la bronchite infectieuse (IBV), du virus responsable de l'anémie du poulet (CAV), du protozoaire Eimeria responsable de la coccidiose, du virus de la maladie de Newcastle (NDV) et du virus de la maladie de Marek (MDV).

6. Virus selon la revendication 5, caractérisé en ce que la protéine hétérologue est choisie parmi les antigènes du virus de la maladie de Gumboro (IBDV), du virus de la bronchite infectieuse (IBV), du virus responsable de l'anémie du poulet (CAV) et du protozoaire Eimeria.

7. Virus selon la revendication 6, caractérisé en ce que la protéine hétérologue est choisie parmi tout ou partie de lecture ouvertes dont la polyprotéine et des parties de la polyprotéine pour l'IBDV, l'antigène E2 pour l'IBV, l'antigène de surface TA4 pour Eimeria, la protéine P50 pour le CAV.

8. Virus selon la revendication 7, caractérisé en ce que la protéine hétérologue est une partie des phases de la polyprotéine pour l'IBDV comprenant les acides aminés 1 à 493 suivis des acides aminés 1010 à 1012.

9. Culture de cellules eucaryotes infectées par un virus de l'Avipox recombinant selon l'une quelconque des revendications 1 à 8.

**10.** Culture de cellules selon la revendication 9 caractérisée en ce qu'il s'agit de cellules aviaires.

**11.** Vaccin caractérisé en ce qu'il contient un virus de l'Avipox selon l'une quelconque des revendications 1 à 8.

**12.** Utilisation d'un virus de l'Avipox selon l'une quelconque des revendications 1 à 8 comme vecteur capable d'exprimer tout ou partie d'une protéine hétérologue au virus.

**13.** Séquence de transfert qui permet d'insérer une séquence d'ADN codant pour tout ou partie d'une protéine hétérologue dans les regions intergéniques du génome du virus de l'Avipox selon l'une quelconque des revendications 1 à 8.

**14.** Plasmide qui porte une séquence selon la revendication 13.

**Claims**

**1.** Recombinant Avipox virus derived from an attenuated strain of Avipox virus and containing, in a nonessential part of its genome, at least one DNA sequence encoding all or part of a protein which is heterologous with respect to the Avipox virus as well as the elements capable of ensuring the expression of this protein inside a cell infected by the said recombinant virus, characterised in that the nonessential part of the genome is composed of a noncoding intergenic region having a sequence of more than 60 nucleotides and situated between two open reading frames (ORF) including their expression signals this intergenic region being chosen from the region, called β1 region, situated between the ORF1 and ORF2 of the TIR (terminal inverted repeats) region and the region, called β2 region, situated between the ORF2 and ORF3 of the TIR region.

**2.** Virus according to Claim 1, characterised in that it is derived from an attenuated strain of Fowlpox virus.

**3.** Virus according to Claim 1 or 2, characterised in that at least one DNA sequence is cloned into the two TIR regions of the virus.

**4.** Virus according to Claim 3, characterised in that a DNA sequence is cloned into the β1 region, inside each of the two TIRs.

**5.** Virus according to any one of Claims 1 to 4, characterised in that the heterologous protein is chosen from the antigens of the infectious bursal disease virus (IBDV), the infectious bronchitis virus (IBV), the virus responsible for chicken anaemia (CAV), the protozoa _Eimeria_ which is responsible for coccidiosis, the Newcastle disease virus (NDV) and the Marek's disease virus (MDV).

**6.** Virus according to Claim 5, characterised in that the heterologous protein is chosen from the antigens of the infectious bursal disease virus (IBDV), the infectious bronchitis virus (IBV), the virus responsible for chicken anaemia (CAV) and the protozoa _Eimeria_.

**7.** Virus according to Claim 6, characterised in that the heterologous protein is chosen from all or part of the open reading frames comprising polyprotein and parts of polyprotein for IBDV, the antigen E2 for IBV, the surface antigen TA4 for _Eimeria_ and the protein P50 for CAV.

**8.** Virus according to Claim 7, characterised in that the heterologous protein is part of the polyprotein phases for IBDV containing the amino acids 1 to 493 followed by the amino acids 1010 to 1012.

**9.** Culture of eukaryotic cells which are infected with a recombinant Avipox virus according to any one of Claims 1 to 8.

**10.** Culture of cells according to Claim 9, characterised in that it relates to fowl cells.

**11.** Vaccine characterised in that it contains an Avipox virus according to any one of Claims 1 to 8.

**12.** Use of an Avipox virus according to any one of Claims 1 to 8 as a vector which is capable of expressing all or part of a protein which is heterologous with respect to the virus.

**13.** Transfer sequence which enables a DNA sequence, encoding all or part of a heterologous protein, to be inserted into the intergenic regions of the genome of the Avipox virus according to any one of Claims 1 to 8.

14. Plasmid carrying a sequence according to Claim 13.

**Patentansprüche**

1. Rekombinantes Avipoxvirus, welches von einem abgeschwächten Avipoxvirusstamm abgeleitet ist und in einem nicht-essentiellen Teil seines Genoms zumindest eine DNS-Sequenz, die für das gesamte oder einen Teil eines mit dem Avipoxvirus heterologen Proteins codiert, sowie die Elemente, die die Expression dieses Proteins in einer durch das rekombinante Virus infizierten Zelle sicherstellen können, enthält, dadurch gekennzeichnet, daß der nicht-essentielle Teil des Genoms aus einer nicht-codierenden Intergen-Region mit einer Sequenz besteht, die mehr als 60 Nucleotide aufweist und zwischen zwei offenen Leserahmen (ORF) angeordnet ist, wobei ihre Expressionssignale eingeschlossen sind, welche Intergen-Region aus der als β1 bezeichneten Region, die zwischen ORF1 und ORF2 der TIR-Region (terminale invertierte Repeats) angeordnet ist, und der als β2 bezeichneten Region, die zwischen ORF2 und ORF3 der TIR-Region angeordnet ist, ausgewählt ist.

2. Virus nach Anspruch 1, dadurch gekennzeichnet, daß es von einem abgeschwächten Fowlpoxvirus abgeleitet ist.

3. Virus nach Anspruch 1 oder 2, dadurch gekennzeichnet, daß zumindest eine DNS-Sequenz in den beiden TIR-Regionen des Virus kloniert ist.

4. Virus nach Anspruch 3, dadurch gekennzeichnet, daß eine DNS-Sequenz in der β1-Region in jeder der beiden TIR kloniert ist.

5. Virus nach einem der Ansprüche 1 bis 4, dadurch gekennzeichnet, daß das heterologe Protein aus den Antigenen des Virus der Gumboro-Krankheit (IBDV), des Virus von infektiöser Bronchitis (IBV), des Virus, das für Hühner-Anämie verantwortlich ist, (CAV), des Protozoons Eimeria, das für Kokzidiose verantwortlich ist, des Newcastle-disease-Virus (NDV) und des Marek-disease-Virus (MDV) ausgewählt ist.

6. Virus nach Anspruch 5, dadurch gekennzeichnet, daß das heterologe Protein aus den Antigenen des Virus der Gumboro-Krankheit (IBDV), des Virus von infektiöser Bronchitis (IBV), des Virus, das für Hühner-Anämie verantwortlich ist, (CAV) und des Protozoons Eimeria ausgewählt ist.

7. Virus nach Anspruch 6, dadurch gekennzeichnet, daß das heterologe Protein aus den gesamten oder einem Teil der offenen Leserahmen ausgewählt ist, darunter aus dem Polyprotein und den Teilen des Polyproteins für IBDV, dem Antigen E2 für IBV, dem Oberflächen-Antigen TA4 für Eimeria, dem Protein P50 für CAV.

8. Virus nach Anspruch 7, dadurch gekennzeichnet, daß das heterologe Protein ein Teil der Rahmen des Polyproteins für IBDV ist, der die Aminosäuren 1 bis 493, gefolgt von den Aminosäuren 1010 bis 1012, umfaßt.

9. Eukaryoten-Zellkultur, welche mit einem rekombinanten Avipoxvirus nach einem der Ansprüche 1 bis 8 infiziert ist.

10. Zellkultur nach Anspruch 9, dadurch gekennzeichnet, daß es sich um Vogel-Zellen handelt.

11. Impfstoff, dadurch gekennzeichnet, daß er einen Avipoxvirus nach einem der Ansprüche 1 bis 8 enthält.

12. Verwendung eines Avipoxvirus nach einem der Ansprüche 1 bis 8 als Vektor, der das gesamte oder einen Teil eines mit dem Virus heterologen Proteins exprimieren kann.

13. Transfersequenz, welche die Insertion einer DNS-Sequenz ermöglicht, die für das gesamte oder einen Teil eines heterologen proteins in den Intergen-Regionen des Genoms des Avipoxvirus nach einem der Ansprüche 1 bis 8 codiert.

14. Plasmid, welches eine Sequenz nach Anspruch 13 trägt.

Fig.1

Fig.2

TIR-R

9.0 kb

TIR-L

6.2 kb

EcoR1

BamH1

EP 0 517 292 B1

Fig.3

39

Fig.4

Fig.5

EP 0 517 292 B1

Fig.6

(235)/NARI
(399)/HINDIII
(405)/SPHI
(411)/PSTI
(417)/SALI
(503)/NCOI
(1003)/BSPMII/BSPEI
(1019)/SACI

12.0  0.0

11.0

1.0

AP

ORF1

(1928)/DRAIII

ORI

(9827)/ECORI

10.0

P75

2.0

(2280)/BCLI
(2433)/NCOI

ORF6

PTIRB1P75LAC.
12063pb

9.0

(8918)/SACII

ORF5

3.0

LACZ

(8367)/SACII

4.0

(3613)/DRAIII
(3774)/BCLI
(3926)/BSSHII

ORF4 ORF3L

8.0

(7827)/PACI

ORF2

(4364)/SACI
(4641)/BSTXI

7.0

6.0

5.0

(5199)/SPLI
(5258)/BSTXI
(5432)/ECORI
(5506)/BAMHI

(6794)/XHOI
(6489)/BSMI
(6477)/SPLI
(6120)/NRUI
(5921)/SPEI

42

EP 0 517 292 B1

EP 0 517 292 B1

Fig.7

Fig.8

A

ORF3  ▷(?)

ORF2  [  ? ⟩

ORF1  [        VP2        |       VP4       |       VP3       ⟩

5' ··· |‐‐‐‐‐‐‐‐‐‐‐‐‐‐‐‐‐‐‐‐‐‐‐‐‐‐‐‐‐‐‐‐‐‐‐‐ ······3'

        500    1000    1500    2000    2500    3000   (pb)

P

0▷    ◁1b

1▷    ◁▷    ◁▷    ◁6

           2 3    4 5

fgt PCR

Bcl1  Pst1  Pst1
▷—|—|—◁ 585pb (0-1b)
  277  74  234

Bcl1      Sac1
▷—————|———◁ 1129pb (1-2)
    760   369

              Pst1
▷—————◁ 670pb (3-4)

        Pst1        Sph1  BamH1
(5-6) 1301pb ▷—————————|———◁
            956    345

EP 0 517 292 B1

EP 0 517 292 B1

|>ORF3        ORF3>|         |>ORF1
MetGluLeuLeuLeuLeuGlnArgTyrHis###        MetThrAsnLeuThrAspGlnThr

          |>ORF2
       MetValSerArgAspGlnThrAsnAspArgSerAspAspLysProAspArgSerAsnP
10  20  30  40  50  60  70  80  90  100
TTGTTCCAGGATGGAACTCCTCCTTCTACAACGCTATCATTGATGGTTAGTAGAGATCAGACAAACGATCGCAGCGATGACAAACCTGACAGATCAAACC
primer 0 5'[CCTCCTTCTACAACGTGATCATTGATGGTTAGTAGAGATCAG]3'  [BCLI]
        [BCLI]     5'[GTAGAGATCAGACAAATGATCACAGCGATGAC]3' primer 1

GlnGlnIleValProPheIleArgSerLeuLeuMetProThrThrGlyProAlaSerIleProAspAspThrLeuGluLysHisThrLeuArgSerGluT
roThrAspCysSerValHisThrGluProSerAspAlaAsnAsnArgThrGlyValHisSerGlyArgHisProGlyGluAlaHisSerGlnValArgAs
110  120  130  140  150  160  170  180  190  200
CAACAGATTGTTCCGTTCATACGGAGCCTTCTGATGCCAACAACCGGACCGGCGTCCATTCCGGACGACACCCTGGAGAAGCACACTCTCAGGTCAGAGA

hrSerThrTyrAsnLeuThrValGlyAspThrGlySerGlyLeuIleValPhePheProGlyPheProGlySerIleValGlyAlaHisTyrThrLeuGl
pLeuAspLeuGlnPheAspCysGlyGlyHisArgValArgAlaAsnCysLeuPheProTrpIleProTrpLeuAsnCysGlyCysSerLeuHisThrAla
210  220  230  240  250  260  270  280  290  300
CCTCGACCTACAATTTGACTGTGGGGGGACACAGGGTCAGGGCTAATTGTCTTTTTCCCTGGATTCCCTGGCTCAATTGTGGGTGCTCACTACACACTGCA

nSerAsnGlyAsnTyrLysPheAspGlnMetLeuLeuThrAlaGlnAsnLeuProAlaSerTyrAsnTyrCysArgLeuValSerArgSerLeuThrVal
GluGlnTrpGluLeuGlnValArgSerAspAlaProAspCysProGluProThrGlyGlnLeuGlnLeuGlnAlaSerGluSerGluSerHisSerG
310  320  330  340  350  360  370  380  390  400
GAGCAATGGGAACTACAAGTTCGATCAGATGCTCCTGACTGCCCAGAACCTACCGGCCAGTTACAACTACTGCAGGCTAGTGAGTCGGAGTCTCACAGTG

ArgSerSerThrLeuProGlyGlyValTyrAlaLeuAsnGlyThrValAsnAlaValThrPheGlnGlySerLeuSerGluLeuThrAspValSerTyrA
                   ORF2>|
luValLysHisThrProTrpTrpArgLeuCysThrLysArgHisArgLysArgArgAspLeuProArgLysProGlu###
410  420  430  440  450  460  470  480  490  500
AGGTCAAGCACACTCCCTGGTGGCGTTTATGCACTAAACGGCACCGTAAACGCCGTGACCTTCCAAGGAAGCCTGAGTGAACTGACAGATGTTAGCTACA

Fig.9a

snGlyLeuMetSerAlaThrAlaAsnIleAsnAspLysIleGlyAsnValLeuValGlyGluGlyValThrValLeuSerLeuProThrSerTyrAspLe

510    520    530    540    550    560    570    580    590    600

ATGGGTTGATGTCTGCAACGGCCAACATCAACGACAAAATTGGGAATGTCCTAGTAGGGGAAGGGGTCACCGTCCTCAGCTTACCCACATCATATGATCT

primer 1b 3' GGAGTCGAATGGGTGTAGTATACTGGA

uGlyTyrValArgLeuGlyAspProIleProAlaIleGlyLeuAspProLysMetValAlaThrCysAspSerSerAspArgProArgValTyrThrIle

610    620    630    640    650    660    670    680    690    700

TGGGTATGTGAGGCTTGGTGACCCCATTCCTGCTATAGGGCTTGACCCAAAAATGGTAGCCACATGTGACAGCAGTGACAGGCCCAGAGTCTACACCATA
ACCC 5'

ThrAlaAlaAspAspTyrGlnPheSerSerGlnTyrGlnProGlyGlyValThrIleThrLeuPheSerAlaAsnIleAspAlaIleThrSerLeuSerI

710    720    730    740    750    760    770    780    790    800

ACTGCAGCCGATGATTACCAATTCTCATCACAGTACCAACCAGGTGGGGTAACAATCACACTGTTCTCAGCCAACATTGATGCTATCACAAGCCTCAGCA

IleGlyGlyGluLeuValPheGlnThrSerValGlnGlyLeuValLeuGlyAlaThrIleTyrLeuIleGlyPheAspGlyThrThrValIleThrArgAl

810    820    830    840    850    860    870    880    890    900

TTGGGGGAGAGCTCGTGTTCCAAACAAGCGTCCAAGGCCTTGTACTGGGCGCTACCATCTACCTTATAGGCTTTGATGGGACTACAGTAATCACCAGAGC

aValAlaSerAspAsnGlyLeuThrAlaGlyThrAspAsnLeuMetProPheAsnLeuValIleProThrAsnGluIleThrGlnProIleThrSerIle

910    920    930    940    950    960    970    980    990    1000

TGTGGCCTCAGACAATGGGCTGACTGCCGGCACCGACAATCTTATGCCATTCAATCTTGTGATTCCGACCAACGAGATAACCCAGCCAATCACATCCATC

LysLeuGluIleValThrSerLysSerGlyGlyGlnAlaGlyAspGlnMetSerTrpSerAlaSerGlySerLeuAlaValThrIleHisGlyGlyAsnT

1010    1020    1030    1040    1050    1060    1070    1080    1090    1100

AAACTGGAGATAGTGACCTCCAAAAGTGGCGGTCAGGCAGGGGACCAGATGTCATGGTCGGCAAGTGGGAGCCTAGCAGTGACAATCCATGGTGGCAACT

Fig.9b

EP 0 517 292 B1

yrProGlyAlaLeuArgProValThrLeuValAlaTyrGluArgValAlaThrGlySerValValThrValAlaGlyValSerAsnPheGluLeuIlePr
1110      1120      1130      1140      1150      1160      1170      1180      1190      1200
ATCCAGGGGCCCTCCGTCCCGTCACACTAGTAGCCTACGAAAGAGTGGCAACAGGATCCGTCGTTACGGTAGCCGGGGTGAGCAACTTCGAGCTGATCCC
                                       primer 2  3′ CCGTTGTCCTAGCCAGCAATGCCAGCGACCCC 5′
                                                                   5′ TTGAGCAACTTCGAGCTGATCCC

oAsnProGluLeuAlaLysAsnLeuValThrGluTyrGlyArgPheAspProGlyAlaMetAsnTyrThrLysLeuIleLeuSerGluArgAspArgLeu
1210      1220      1230      1240      1250      1260      1270      1280      1290      1300
AAATCCTGAACTAGCAAAGAACCTGGTTACAGAATACGGCCGATTTGACCCAGGAGCCATGAACTACACAAAATTGATACTGAGTGAGAGGGACCGTCTT
AAATCCTG 3′ primer 3

GlyIleLysThrValTrpProThrArgGluTyrThrAspPheArgGluTyrPheMetGluValAlaAspLeuAsnSerProLeuLysIleAlaGlyAlaP
1310      1320      1330      1340      1350      1360      1370      1380      1390      1400
GGCATCAAGACCGTCTGGCCAACAAGGGAGTACACTGACTTTCGTGAGTACTTCATGGAGGTGGCCGACCTCAACTCTCCCCTGAAGATTGCAGGAGCAT

heGlyPheLysAspIleIleArgAlaIleArgArgIleAlaValProValValSerThrLeuPheProProAlaAlaProLeuAlaLeuAlaIleGlyGl
1410      1420      1430      1440      1450      1460      1470      1480      1490      1500
TTGGCTTCAAAGACATAATCCGGGCCATAAGGAGGATAGCTGTGCCGGTGGTCTCTACATTGTTCCCACCTGCAGCTCCCCTAGCCCTTGCAATTGGGGA

uGlyValAspTyrLeuLeuGlyAspGluAlaGlnAlaAlaSerGlyThrAlaArgAlaAlaSerGlyLysAlaArgAlaAlaSerGlyArgIleArgGln
1510      1520      1530      1540      1550      1560      1570      1580      1590      1600
AGGTGTAGACTACCTGCTGGGCGATGAGGCACAGGCTGCTTCAGGAACTGCTCGAGCCGCGTCAGGAAAAGCAAGAGCTGCCTCAGGCCGTATAAGGCAG

LeuThrLeuAlaAlaAspLysGlyTyrGluValValAlaAsnLeuPheGlnValProGlnAsnProValValAspGlyIleLeuAlaSerProGlyValL
1610      1620      1630      1640      1650      1660      1670      1680      1690      1700
CTAACTCTCGCCGCTGACAAGGGGTACGAGGTTGTCGCGAATCTATTCCAGGTGCCCCAGAATCCCGTAGTCGACGGGATTCTTGCTTCACCTGGGGTAC

euArgGlyAlaHisAsnLeuAspCysValLeuArgGluGlyAlaThrLeuPheProValValIleThrThrValGluAspAlaMetThrProLysAlaLe
1710      1720      1730      1740      1750      1760      1770      1780      1790      1800
TCCGCGGCGCACATAACCTCGACTGCGTGCTAAGAGAGGGTGCCACGCTATTCCCTGTGGTCATTACGACAGTGGAAGACGCCATGACACCCAAAGCACT

Fig.9c

uAsnSerLysMetPheAlaVallleGluGlyValArgGluAspLeuGlnProProSerGlnArgGlySerPhelleArgThrLeuSerGlyHisArgVal

1810    1820    1830    1840    1850    1860    1870    1880    1890    1900

GAACAGCAAAATGTTTGCTGTCATTGAAGGCGTGCGAGAAGACCTGCAGCCTCCATCTCAAAGAGGATCATTCATACGAACTCTCTCCGGACACAGAGTC

primer 4  3′ CGACAGTAACTTCCGCACGCTCTTCTGGACGTC 5′

5′ CTGCAGCCTCCATCTCAAAGAGGATCATTCATACGAACTC 3′ primer 5

TyrGlyTyrAlaProAspGlyValLeuProLeuGluThrGlyArgAspTyrThrValValProlleAspAspValTrpAspAspSerlleMetLeuSerL

1910    1920    1930    1940    1950    1960    1970    1980    1990    2000

TATGGATATGCTCCAGATGGGGTACTTCCACTGGAGACTGGGAGAGACTACACCGTTGTCCCAATAGATGATGTATGGGACGACAGCATTATGCTGTCCA


ysAspProlleProProlleValGlyAsnSerGlyAsnLeuAlalleAlaTyrMetAspValPheArgProLysValProlleHisValAlaMetThrGl

2010    2020    2030    2040    2050    2060    2070    2080    2090    2100

AAGACCCCATACCTCCTATTGTGGGAAACAGTGGCAACCTAGCCATAGCTTACATGGATGTGTTTCGACCCAAAGTTCCCATCCATGTGGCCATGACAGG


yAlaLeuAsnAlaCysGlyGlulleGluLysValSerPheArgSerThrLysLeuAlaThrAlaHisArgLeuGlyLeuLysLeuAlaGlyProGlyAla

2110    2120    2130    2140    2150    2160    2170    2180    2190    2200

AGCCCTCAATGCTTGTGGCGAGATTGAGAAAGTAAGCTTTAGAAGCACCAAGCTCGCTACTGCACACCGACTTGGCCTCAAGTTGGCTGGTCCCGGAGCA


PheAspValAsnThrGlyProAsnTrpAlaThrPhelleLysArgPheProHisAsnProArgAspTrpAspArgLeuProTyrLeuAsnLeuProTyrL

2210    2220    2230    2240    2250    2260    2270    2280    2290    2300

TTCGATGTAAACACCGGGCCTAATTGGGCAACGTTCATCAAACGTTTCCCTCACAATCCACGCGACTGGGACAGGCTCCCCTACCTCAACCTTCCATACC


euProProAsnAlaGlyArgGlnTyrHisLeuAlaMetAlaAlaSerGluPheLysGluThrProGluLeuGluSerAlaValArgAlaMetGluAlaAl

2310    2320    2330    2340    2350    2360    2370    2380    2390    2400

TTCCACCCAATGCAGGACGCCAGTACCACCTTGCCATGGCTGCATCAGAGTTCAAAGAGACCCCTGAACTCGAGAGCGCCGTCAGAGCCATGGAAGCAGC


aAlaAsnValAspProLeuPheGlnSerAlalleSerValPheMetTrpLeuGluGluAsnGlylleValThrAspMetAlaAsnPheAlaLeuSerAsp

2410    2420    2430    2440    2450    2460    2470    2480    2490    2500

AGCCAACGTGGACCCACTATTCCAATCTGCAATCAGTGTGTTCATGTGGCTGGAAGAGAATGGGATTGTGACTGACATGGCCAACTTCGCACTCAGCGAC

Fig.9d

ProAsnAlaHisArgMetArgAsnPheLeuAlaAsnAlaProGlnAlaGlySerLysSerGlnArgAlaLysTyrGlyThrAlaGlyTyrGlyValGluA

CCGAACGCCCATCGGATGCGAAATTTTCTTGCAAACGCACCACAAGCAGGCAGCAAGTCGCAACGGGCCAAGTACGGGACAGCAGGCTACGGAGTGGAGG

laArgGlyProThrProGluGluAlaGlnArgGluLysAspThrArgIleSerLysLysMetGluThrMetGlyIleTyrPheAlaThrProGluTrpVa

CCCGGGGCCCCACACCAGAGGAAGCACAGAGGGAAAAAGACACACGGATCTCAAAGAAGATGGAGACCATGGGCATCTACTTTGCAACACCAGAATGGGT

lAlaLeuAsnGlyHisArgGlyProSerProGlyGlnLeuLysTyrTrpGlnAsnThrArgGluIleProAspProAsnGluAspTyrLeuAspTyrVal

AGCACTCAATGGGCACCGAGGGCCAAGCCCTGGCCAGCTAAAGTACTGGCAGAACACACGAGAAATACCGGACCCAAACGAGGACTATCTAGACTACGTG

HisAlaGluLysSerArgLeuAlaSerGluGluGlnIleLeuLeuArgAlaAlaThrSerIleTyrGlyAlaProGlyGlnAlaGluProProGlnAlaPheI

CATGCAGAGAAGAGCCGGTTGGCATCAGAAGAACAAATCCTAAGGGCAGCTACGTCGATCTACGGGGCTCCAGGACAGGCAGAGCCACCCCAAGCTTTCA

leAspGluValAlaLysValTyrGluIleAsnHisGlyArgGlyProAsnGlnGluGlnMetLysAspLeuLeuLeuThrAlaMetGluMetLysHisAr

TAGACGAAGTTGCCAAAGTCTATGAAATCAACCATGGACGTGGCCCAAACCAAGAGCAGATGAAAGATCTGCTCTTGACTGCGATGGAGATGAAGCATCG

gAsnProArgArgAlaProProLysProLysProLysProAsnAlaProThrProArgProProGlyArgLeuGlyArgTrpIleArgThrValSerAsp

CAATCCCAGGCGGGCTCCACCAAAGCCCAAGCCAAAACCCAATGCTCCAACACCGAGACCCCCTGGTCGGCTGGGCCGCTGGATCAGGACTGTCTCTGAT

ORF1>I
GluAspLeuGlu###

GAGGACCTTGAGTGA

primer 6 3´ [CCGAGGACCCTCCTAGGGCTGTGGTGGGCG] 5´

BAMHI

Fig.9e

EP 0 517 292 B1

Fig.10

Fig.11

## FIG. 12A

## FIG. 12B

Figure 13 A

| V8 | V17 | V16 | V15 | V14 | V12 | V11 | IBDV | MW |
|----|-----|-----|-----|-----|-----|-----|------|-----|

Kd

69

46 VPX

VP2

VP3

30 VP4

21.5

Figure 13 B

| V8 | V17 | V16 | V15 | V14 | V12 | V11 | IBDV | MW |
|----|-----|-----|-----|-----|-----|-----|------|-----|

Kd

69

46

VP3

30

21.5